(19) Europäisches Patentamt
European Patent Office
Office européen des brevets

(11) EP 1 478 780 B1

(12) FASCICULE DE BREVET EUROPEEN

(45) Date de publication et mention de la délivrance du brevet:
**09.01.2008 Bulletin 2008/02**

(21) Numéro de dépôt: **03722673.5**

(22) Date de dépôt: **25.02.2003**

(51) Int Cl.:
***C12Q 1/68*** *(2006.01)* ***G06F 19/00*** *(2006.01)*

(86) Numéro de dépôt international:
**PCT/FR2003/000609**

(87) Numéro de publication internationale:
**WO 2003/072823 (04.09.2003 Gazette 2003/36)**

(54) **PROCEDE DE DETECTION IN VITRO DES CANCERS PAR LA MISE EN EVIDENCE DE DESEQUILIBRES ALLELIQUES DE MARQUEURS D INSERTION-DELETI ON**

VERFAHREN ZUR DETEKTION VON KREBSERKRANKUNGEN DURCH DEN NACHWEIS VON ALLELISCHEN UNGLEICHGEWICHTEN VON INSERTIONS-DELETIONS-MARKERN

METHOD FOR IN VITRO DETECTION OF CANCERS BY HIGHLIGHTING ALLELIC IMBALANCES IN INSERTION/DELETION MARKERS

(84) Etats contractants désignés:
**AT BE BG CH CY CZ DE DK EE ES FI FR GB GR HU IE IT LI LU MC NL PT SE SI SK TR**

(30) Priorité: **25.02.2002 FR 0202380**

(43) Date de publication de la demande:
**24.11.2004 Bulletin 2004/48**

(60) Demande divisionnaire:
**07020272.6**

(73) Titulaire: **ASSISTANCE PUBLIQUE, HOPITAUX DE PARIS
75004 Paris (FR)**

(72) Inventeurs:
- **GRANDCHAMP, Bernard
F-75017 Paris (FR)**
- **MENTRE, France
F-75006 Paris (FR)**

(74) Mandataire: **Noel, Chantal Odile et al
Cabinet Orès
36, rue Saint Pétersbourg
75008 Paris (FR)**

(56) Documents cités:
**WO-A-00/31306**

- **DOMINIQUE S ET AL: "Evaluation of new DNA markers for screening of bladder cancer by allelic imbalance analysis in urine sediment" EUROPEAN UROLOGY SUPPLEMENTS, vol. 1, no. 1, January 2002 (2002-01), page 78, XP008012287 ISSN: 1569-9056**
- **WADA T ET AL: "Bladder cancer: allelic deletions at and around the retinoblastoma tumor suppressor gene in relation to stage and grade." CLINICAL CANCER RESEARCH: AN OFFICIAL JOURNAL OF THE AMERICAN ASSOCIATION FOR CANCER RESEARCH. UNITED STATES FEB 2000, vol. 6, no. 2, February 2000 (2000-02), pages 610-615, XP002250012 ISSN: 1078-0432**
- **SCHNEIDER ANNE ET AL: "Evaluation of microsatellite analysis in urine sediment for diagnosis of bladder cancer." CANCER RESEARCH, vol. 60, no. 16, 15 August 2000 (2000-08-15), pages 4617-4622, XP002227526 ISSN: 0008-5472**
- **PRIMDAHL HANNE ET AL: "Allelic imbalances in human bladder cancer: genome-wide detection with high-density single-nucleotide polymorphism arrays." JOURNAL OF THE NATIONAL CANCER INSTITUTE. UNITED STATES 6 FEB 2002, vol. 94, no. 3, 6 February 2002 (2002-02-06), pages 216-223, XP008012556 ISSN: 0027-8874**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

• GHEBRANIOUS N ET AL: "Human diallelic insertion/deletion polymorphisms." AMERICAN JOURNAL OF HUMAN GENETICS, vol. 67, no. 4 Supplement 2, October 2000 (2000-10), page 34, XP002226840 50th Annual Meeting of the American Society of Human Genetics; Philadelphia, Pennsylvania, USA; October 03-07, 2000 ISSN: 0002-9297

## Description

La présente invention relève du domaine médical, et intéresse en particulier les unités et services hospitaliers et cliniques de cancérologie, ainsi que les laboratoires d'analyses médicales. L'invention concerne en effet le diagnostic non invasif et/ou le pronostic et/ou le suivi de l'efficacité d'un traitement anticancéreux.

A cette fin, l'invention met en oeuvre des techniques de biologie moléculaire pour l'analyse *in vitro* de prélèvements biologiques, associées à une méthode statistique de traitement des données issues de ladite analyse, cette combinaison de la biologie moléculaire et la statistique permettant de déceler des anomalies génétiques présentes dans les tumeurs de patients atteints de cancer.

Ainsi, l'invention a pour objet un procédé de détection des cellules tumorales contenues dans un prélèvement biologique par mise en évidence de déséquilibres alléliques de marqueurs chromosomiques d'insertion-délétion, ledit procédé faisant appel à l'amplification multiplex desdits marqueurs par réaction en chaîne thermo-dépendante, au calcul d'un score statistique global, sur l'ensemble des marqueurs étudiés, et à la comparaison dudit score par rapport au seuil de normalité fixé.

D'un point de vue épidémiologique, les tumeurs de vessie (TV) représentent une affection très fréquente. Les TV figurent notamment en $5^{ème}$ position des cancers les plus fréquents en occident, où elles sont responsables de 3 % de l'ensemble des décès liés au cancer. Aux Etats-Unis, les TV représentent respectivement le $4^{ème}$ et $8^{ème}$ cancer le plus fréquent chez l'homme et la femme, avec 53000 nouveaux cas par an et une mortalité évaluée à 12000 cas par an (1, 2). En France, comme dans l'ensemble des pays industrialisés, l'incidence des TV est actuellement en augmentation. Ainsi est-elle passée de 13 à 17,4/100000 par an entre 1980 et 1994 (3). En 1990, le nombre de décès en France imputés aux TV était d'environ 7/100000 habitants, soit 5 % de l'ensemble des décès par cancer.

Histologiquement, les TV correspondent, pour 95 % d'entre elles, à des carcinomes à cellules transitionnelles. En France, les carcinomes épidermoïdes, ainsi que les adénocarcinomes restent rares.

La classification de l'UICC (Union Internationale Contre le Cancer) a défini différents stades d'envahissement de la paroi vésicale, ainsi que divers grades cytologiques. Cette classification se trouve résumée dans les tableaux I et II suivants.

**Tableau I**

| | Stade histologique | | | | | | |
|---|---|---|---|---|---|---|---|
| Envahissement | Carcinome *in situ* | pTa | pT1 | pT2 | pT3a | pT3b | pT4 /b |
| urothélium | + | + | + | + | + | + | + |
| chorion | - | - | + | + | + | + | + |
| muscle superficiel | - | - | - | + | + | + | + |
| muscle profond | - | - | - | - | + | + | + |
| graisse périvésicale | - | - | - | - | - | + | + |
| organes voisins | - | - | - | - | - | - | + |

**Tableau II**

| Grade histologique | Atypies nucléaires | Activité mitotique | Différenciation |
|---|---|---|---|
| G1 | + | + | cellule bien différenciée |
| G2 | ++ | ++ | cellule moyennement différenciée |
| G3 | +++ | +++ | cellule peu différenciée |

D'un point de vue symptomatique, l'hématurie et les signes fonctionnels urinaires irritatifs (pollakiurie, impériosités, fuites par impériosité) représentent l'essentiel des manifestations cliniques des TV. L'on observe plus rarement des complications liées à la lésion (obstructions urétérales, phénomènes de compression pelvienne) ou bien à sa dissémination (métastases à distance).

Au moment du diagnostic initial, environ 70 à 80 % des tumeurs sont classées superficielles (i.e., n'envahissant pas le muscle vésical), et 20 à 30 % invasives. Parmi ces dernières, plus de la moitié présentent dès le premier diagnostic un envahissement ganglionnaire ou systémique.

Après résection trans-urétrale éventuellement associée à un traitement adjuvant (immunothérapie ou chimiothérapie endovésicale), l'histoire naturelle des lésions superficielles est marquée par deux risques majeurs, que sont la récidive et la progression.

Il apparaît donc nécessaire, à la lumière de leur importance tant en termes épidémiologiques qu'évolutifs, de surveiller les TV superficielles avec vigilance.

Cette surveillance peut être effectuée par différentes méthodes, lesquelles se révèlent plus ou moins adaptées en fonction des cas. Elles sont parfois insuffisantes à elles seules, ne sont alors utiles que combinées, et présentent, en tout état de cause, un certain nombre d'avantages et inconvénients sommairement rappelés ci-après.

L'examen clinique, bien que rarement contributif, s'attache à la recherche de survenue d'hématuries (macroscopiques ou microscopiques dépistées grâce aux bandelettes urinaires), et à l'apparition de troubles mictionnels irritatifs (pollakiurie, impériosités). Enfin, les touchers pelviens permettent de déceler une infiltration de la base vésicale, signe du caractère infiltrant d'une tumeur.

La cytoscopie est réalisée à l'aide d'un cystoscope rigide ou d'un fibroscope souple. Cet examen, certes performant, est invasif, parfois mal toléré et peut être source de complications (sténoses de l'urètre, infections urinaires). De plus, la cytoscopie ne permet pas toujours une bonne exploration de la face antérieure de la vessie, pas plus que des régions intradiverticulaires.

Le cytodiagnostic urinaire a ce double avantage d'être à la fois simple et non invasif. Sa sensibilité globale, c'est-à-dire sa capacité à déceler l'anomalie lorsqu'il y a lieu, reste néanmoins faible (entre 40 et 60 %) (4, 5). En effet, cette sensibilité dépend dans une large mesure de l'anatomopathologiste effectuant l'examen étant donné qu'une part de subjectivité intervient inéluctablement dans l'interprétation finale. En outre, sensibilité et spécificité du cytodiagnostic urinaire varient considérablement selon le grade tumoral, la spécificité désignant ici l'aptitude de la méthode à conclure à juste titre à l'absence d'anomalies. Ainsi, dans le cas de lésions de haut grade (G3, *cf.* Tableau II, supra), la sensibilité est évaluée à 90 % et la spécificité à 98 % (6). En revanche, la sensibilité chute entre 11 et 25 % s'agissant des tumeurs de bas grade (G1, *ibid.*) (6).

Une autre méthode de surveillance des TV consiste à doser la Nuclear Matrix Protein 22 (Protéine 22 de la Matrice Nucléaire, NMP22). Cette protéine joue un rôle dans l'organisation du fuseau mitotique. En l'espèce, elle est dosée dans les urines grâce à un test immuno-enzymatique quantitatif. En fonction des limites de positivité choisies, la sensibilité demeure très variable (7). Néanmoins, la plupart des études retrouvent des sensibilités et spécificités de 60 à 70 % (8). L'avantage de ce test réside dans le fait que sa sensibilité ne dépend pas du grade tumoral.

Le « BTA TRAK Test » utilise des anticorps monoclonaux dirigés contre le facteur H du complément humain, dont le taux urinaire est corrélé à la présence de TV. Il s'agit là d'un test quantitatif, dont la sensibilité oscille entre 62 et 77 % et reste fortement liée au grade (G1 : 48 % ; G2 : 59 % ; et G3 : 88 %) (8, 9). En revanche, sa spécificité est plus faible : entre 48 et 65 % selon les études. Ceci est dû au fait que le test réagit non seulement en cas de lésions tumorales, mais également avec le facteur H sanguin en cas d'hématuries. Aussi toutes les circonstances susceptibles d'entraîner une hématurie peuvent-elles être sources de faux-positifs (8).

L'acide hyaluronique (HA) est un glycosaminoglycane dégradé par la hyaluronidase (HAase) en petits fragments impliqués dans l'adhésion cellulaire et l'angiogénèse. Dans le cadre du test HA-HAase, les taux de ces deux éléments sont dosés dans les urines selon une technique ENLISA. Une sensibilité de l'ordre de 90 % (indépendante des grades et stades tumoraux) et une spécificité de 84 % ont été rapportées (8, 10).

L'« immunocyt » consiste en une méthode combinant cytologie urinaire classique et test en immunofluorescence, ledit test utilisant trois anticorps monoclonaux dirigés contre deux mucines et une forme de l'enzyme de conversion de l'angiotensine (ECA). La sensibilité de cette méthode combinatoire est de 90 % (indépendamment du grade tumoral) et sa spécificité de 98 % chez des témoins sains. En revanche, la spécificité chute considérablement chez les patients porteurs d'affections urologiques bénignes : 85 % en cas de microhématurie, 60 % en cas de cystite et 50 % en cas d'adénome prostatique (8, 11).

Initialement explorée pour la mise en évidence de l'activité télomérasique *in vitro* (kit PCR-ELISA), les dernières versions de la méthode de dosage de ladite activité sont fondées sur une quantification des ARN messagers (ARNm) codant la télomérase par RT-PCR (transcription inverse couplée à la réaction de polymérisation en chaîne). La sensibilité de cette technique varie de 0 à 83 % ; sa spécificité est de l'ordre de 70 % (12, 13). La spécificité peut cependant chuter en cas de processus inflammatoire siégeant au niveau de l'appareil urinaire (expression de la télomérase par les lymphocytes activés). Par ailleurs, l'existence d'une hématurie macroscopique peut entraîner la détection de faux-négatifs. Enfin, la technique de RT-PCR nécessite un traitement quasi extemporané des urines (12).

Il convient de noter que de nombreux autres marqueurs utiles pour la détection des TV sont actuellement en cours de développement (cytokératine 20, variants de CD44, NMP de type BLCA-4, uroplaquine...).

Cependant, pour l'heure, aucune méthode de surveillance et détection des TV ne se distingue véritablement pour ses « performances », en termes de sensibilité et spécificité.

Dans un tel contexte, la recherche d'anomalies génétiques permettant une détection à la fois fiable et non invasive desdites TV représente un enjeu important.

Les tumeurs se forment par accumulation de mutations génétiques qui altèrent la fonction de gènes spécifiques, à savoir les oncogènes et les gènes suppresseurs de tumeurs. Cette accumulation peut être accélérée par une instabilité chromosomique importante, laquelle se traduit par la perte d'un ou plusieurs chromosomes entiers, ou de partie(s) seulement d'un chromosome, voire la duplication d'un chromosome, ou encore une disomie uniparentale (i.e., la perte d'un chromosome puis la duplication du chromosome restant).

La comparaison, chez un individu donné, de l'acide nucléique issu du tissu normal (par exemple, l'ADN leucocytaire) et du tissu tumoral peut permettre la mise en évidence d'une modification ou perte de matériel chromosomique et, par là-même, révéler un processus tumoral.

Un individu hétérozygote pour un marqueur chromosomique polymorphe présente une copie de chaque allèle dudit marqueur. Le rapport théorique entre ces deux allèles est alors de 1 (c'est-à-dire 1/1).

Certaines anomalies génétiques sont susceptibles de modifier ce rapport. Par définition, les « déséquilibres alléliques » (DA) désignent toute modification du rapport entre les copies alléliques d'un marqueur.

Les anomalies génétiques peuvent être de plusieurs natures. Il peut s'agir de : (i) une augmentation du nombre de chromosomes (par exemple, une trisomie faisant passer le rapport de 1/1 à 2/1) ; (ii) une délétion plus ou moins étendue de la région chromosomique contenant l'allèle ; (iii) une monosomie (passage du rapport 1/1 à 1/0) ; ou une disomie uniparentale acquise (passage du rapport 1/1 à 2/0).

Les microsatellites sont de courtes séquences d'ADN composées de mono-, di-, tri-, ou tétranucléotides, lesdites séquences étant répétées N fois (N allant de 10 à 60). Environ 100000 séquences microsatellites seraient distribuées de façon aléatoire et homogène dans le génome humain (14). Ces séquences sont le plus souvent situées dans des régions non codantes, bien qu'elles puissent être également présentes dans les introns, voire les exons des gènes.

Les microsatellites se caractérisent par une grande stabilité individuelle, stabilité telle que chacun possède une distribution allélique de microsatellites qui lui est propre. Nonobstant cette stabilité à l'échelle de l'individu, les microsatellites sont hautement polymorphes entre plusieurs individus. A cet égard, lesdits microsatellites sont multialléliques, un grand nombre d'allèles différents, fonction du nombre de répétitions, pouvant exister. En outre, leur taux d'hétérorygotie varie de 70 à 95 %.

En particulier, s'agissant des TV, l'on trouve répertorié dans la littérature un grand nombre de cas d'anomalies génétiques, identifiées comme étant à l'origine de DA.

Ainsi, des DA ont été mis en évidence dans les TV à l'aide de marqueurs microsatellites dès 1994 (15). Ces marqueurs ont du reste été proposés comme examen à visée diagnostique à partir de 1996 (16). Depuis lors, plusieurs équipes ont rapporté des résultats issus du diagnostic (17 ; 18 ; 19), ou du suivi (20) des TV au moyen de marqueurs microsatellites.

Dans ces études, les sensibilités s'étendaient de 84 à 91 %, et les spécificités de 85 à 100 %, et ce, en utilisant entre 10 et 20 marqueurs microsatellites.

Néanmoins, l'utilisation de ce type de marqueurs aux fins de la détection de DA présentent deux inconvénients majeurs.

D'une part, d'un point de vue technique, il est très difficile, voire impossible, de coamplifier au sein d'une même réaction plusieurs séquences microsatellites telles que chacune serait représentative d'un marqueur donné. En effet, comme précédemment indiqué, les séquences microsatellites sont répétées à l'identique un grand nombre de fois et peuvent être communes à différents marqueurs. Ceci rend techniquement difficile, voire impossible, l'amplification simultanée au cours d'une même réaction de PCR de plusieurs de ces marqueurs. En effet, les amorces ne peuvent en pratique pas être dessinées pour n'amplifier qu'une copie d'une séquence microsatellite, sans que ne soient amplifiées simultanément d'autres copies répétées au sein du même marqueur, ainsi que des copies de cette même séquence appartenant à d'autres marqueurs. En conséquence, chaque marqueur microsatellite doit être amplifié individuellement. Dans la mesure où, pour un individu donné, au moins une dizaine de marqueurs microsatellites doivent être étudiés, le recours à des tests utilisant lesdits marqueurs non seulement est coûteux en temps et réactifs, mais également consomme de grandes quantités d'ADN, ce qui peut représenter une limitation supplémentaire s'opposant à une mise en oeuvre en routine.

D'autre part, le principe de tels tests repose sur la mise en évidence d'une modification du rapport de surface des signaux correspondant aux produits d'amplification des deux allèles d'un microsatellite (Figure 1A). Brièvement, l'ADN est amplifié et le rapport de surface des signaux mesuré, s'agissant du prélèvement biologique à analyser, par exemple d'origine urinaire lorsqu'il est question des TV, et d'un prélèvement de référence, sanguin par exemple. Pour conclure à l'existence d'un DA, il convient de mettre en évidence une différence significative entre les rapports de surface des signaux relatifs aux deux prélèvements. Pour ce faire, il faut, le cas échéant, que la différence observée puisse être imputée aux fluctuations expérimentales et, dans tous les autres cas, qu'elle puisse être interprétée à juste titre comme reflétant la présence ou l'absence d'un DA. En l'espèce, s'agissant des microsatellites, l'expérimentateur ne peut contrôler les biais d'amplification sources d'interprétations erronée, le prélèvement de référence conduisant lui-même à un rapport différent de 1 (1/1) pour un individu hétérozygote. En outre, les rapports en question varient d'un individu à l'autre, dans la mesure où, comme indiqué supra, chaque individu possède deux allèles particuliers d'un microsatellite, parmi un grand nombre possible. Aussi les bornes de significativité ne peuvent-elles avoir un sens véritable que pour un seul individu. Elles sont donc choisies empiriquement par l'expérimentateur, comme en témoigne les différences observées dans la littérature, selon les auteurs.

Schneider et al (Cancer Research (2000) 60: 4617-4622) décrivent un procédé de détection de cellules tumorales contenues dans un prélèvement biologique par mise en évidence des déséquilibres alléliques de marqueurs microsatellites comprenant l'amplification des marqueurs par PCR quantitative, le calcul du rapport R de hauteur des deux pics correspondant aux deux allèles de chaque marqueur, la comparaison du rapport R (Ub/Ua) avec un rapport Rf (Bb/Ba) obtenu à partir d'un échantillon de référence pour déterminer l'intensité du déséquilibre allélique et la comparaison de la valeur de l'intensité du déséquilibre allélique obtenue avec un intervalle de normalité.

Pour obvier à ces inconvénients majeurs, à la fois en termes de faisabilité et d'interprétation des résultats, la présente invention utilise, aux fins de la mise en évidence des DA, des marqueurs autres que les microsatellites usuels, que sont les marqueurs polymorphes bi-alléliques d'insertion-délétion, ou SIDP pour « Short Insertion Deletion Polymorphism ».

Les SIDP se caractérisent par la présence ou l'absence de quelques nucléotides. Si l'on prend pour exemple la séquence suivante : ...ATTTGCGCTAA**GCTAGCTA**TTAT..., elle correspond à un SIDP si elle est susceptible de contenir une ou deux copies de la séquence courte GCTA.

Les SIDP sont, par définition, bi-alléliques, puisqu'il existe seulement deux allèles différents d'un même marqueur. En conséquence, l'individu peut être homozygote pour l'un des deux allèles, ou hétérozygote, possédant alors les deux allèles différents possibles (Figure 1 B).

Le taux d'hétérozygotie des SIDP est plus faible que celui des microsatellites. En particulier, ce taux est de l'ordre de 40 % pour les marqueurs ayant servi à l'étude des TV (cf. Exemples ci-après).

Des SIDP ont été identifiés et recensés par l'équipe du Docteur Weber, au « Center for Medical Genetics » de Marshfield (Wisconsin). Les séquences des SIDP sont disponibles sur le site : http://research.marshfieldclinic.org.

L'invention consiste donc en un nouveau test de dépistage des tumeurs, notamment des tumeurs de la vessie, ledit test reposant sur la mise en évidence de DA dans les cellules contenues dans un prélèvement biologique, urinaire par exemple, à l'aide de marqueurs bi-alléliques du type SIDP.

Ainsi, la présente invention a pour objet un procédé de détection de cellules tumorales contenues dans un prélèvement biologique par mise en évidence des déséquilibres alléliques d'au moins quinze, et de préférence au trente marqueurs chromosomiques bi-alléliques d'insertion-délétion, ledit procédé comprenant au moins les étapes suivantes :

a) l'amplification quantitative multiplex desdits marqueurs ;

b) le calcul du rapport R de hauteur des deux pics correspondant aux signaux d'amplification obtenus pour les deux allèles de chaque marqueur ;

c) le calcul d'un score statistique global, relatif à l'ensemble desdits marqueurs, qui se distribue selon une loi du khi-deux dont le nombre de degrés de liberté est égal au nombre de marqueurs testés, ledit score global étant calculé selon la formule :

$$\text{Score} = \Sigma\, d_i^2 = \Sigma\, \{[\text{LnR}_i - m(\text{LnR}_f)] \div s(\text{LnRf})\}^2,$$

où :

$d_i$ désigne une distance statistique, calculée pour un marqueur i ;

$R_i$ désigne, pour chaque marqueur, le rapport de hauteur des deux pics calculé à partir du prélèvement biologique ;

$R_f$ désigne, pour chaque marqueur, le rapport de hauteur des deux pics calculé à partir d'un échantillon de référence ;

$m(\text{LnR}_f)$ correspond à la moyenne des valeurs de $\text{LnR}_f$ ; et

$s(\text{LnR}_f)$ correspond à l'écart type des valeurs de $\text{LnR}_f$ ; et

d) la comparaison dudit score par rapport au seuil de normalité fixé, au-delà duquel le prélèvement biologique est considéré comme pathologique, ledit seuil de normalité correspondant à la valeur du khi-deux pour une valeur choisie du risque de première espèce $\alpha$ et le nombre de marqueurs testés.

Selon l'invention, les termes « procédé », « méthode » et « test » sont équivalents.

Au sens de la présente invention, la « détection » de cellules tumorales s'entend de la mise en évidence de telles cellules. Ladite « détection » est utile au diagnostic non invasif de tumeurs, et/ou au pronostic après identification de tumeurs, et/ou au suivi d'un traitement anticancéreux.

Les termes « cancer » et « tumeur » sont, conformément à l'usage médical, équivalents.

Selon l'acception usuelle dans le domaine médical, un « prélèvement biologique », ou un « échantillon biologique », ou encore un « échantillon » désigne tout élément extrait d'un corps vivant, en particulier du corps humain, tel que le sang, les biopsies, urines, crachats, ledit élément contenant des cellules et étant utile notamment à des fins de diagnostic.

L'on entend par « marqueur », une séquence particulière d'acide nucléique, permettant une détection et un suivi tout à fait spécifiques des DA au sein d'un échantillon contenant des cellules. Ledit marqueur peut être assimilé à un « traceur » de DA dans ces cellules. Pour ce faire, encore faut-il que le marqueur soit informatif, c'est-à-dire, en l'occurrence, présenter un taux d'hétérozygotie aussi élevé que possible, ceci afin de fournir un élément de comparaison susceptible d'être exploité, par exemple en termes de différence entre les signaux représentant les produits d'amplification des deux allèles dudit marqueur (Figure 1 B).

Par référence à l'étape a) du procédé de détection sus-visé, le terme « amplification » est revêtu d'un sens générique, en ce qu'il englobe toute technique de biologie moléculaire dont le principe consiste à amplifier ou multiplier une séquence d'acide nucléique cible. L'on peut citer à titre d'exemples non limitatifs, la PCR, l'amplification par transcription (Transcription-Mediated Amplification ; TMA), l'amplification basée sur des séquences d'acides nucléiques (Nucleic Acid Sequence Based Amplification ; NASBA), l'auto-réplication de séquences (Self-Sustained Sequence Replication ; 3SR), l'amplification par déplacement de brin (Strand Displacement Amplification ; SDA) et la réaction de ligature en chaîne (Ligase Chain Reaction ; LCR).

Les réactions d'amplification ainsi visées peuvent être isothermes ou nécessiter l'exposition cyclique à différentes températures. Selon cette dernière configuration, l'on parle avantageusement de réaction d'amplification « thermo-dépendante », bien que ce dernier terme puisse parfois être tacite, auquel cas l'homme du métier est à même de le déduire clairement et sans ambiguïté du contexte.

D'après le vocabulaire usuel en matière de PCR, les séquences d'acides nucléiques à amplifier sont dites « cibles » ou « matrices ». Dans le contexte particulier de la présente invention, lesdites séquences correspondent à des marqueurs tels que définis supra et, plus particulièrement, des marqueurs chromosomiques.

L'analyse effectuée conformément au procédé selon l'invention est « quantitative », en ce qu'elle permet la quantification des produits d'amplification correspondant aux deux allèles d'un même marqueur, et fournit par là-même la base du calcul du rapport de hauteur des pics relatifs auxdits allèles [étape b) du procédé], ainsi que l'élément à comparer selon l'étape c) dudit procédé. Par exemple, l'une des amorces de chacun des couples utilisés lors de l'étape a) peut être marquée à l'aide d'un fluorochrome.

L'étape a) du procédé objet de l'invention concerne les réactions d'amplification « multiplex » dans la mesure où se déroulent non pas une réaction d'amplification à l'aide d'un couple d'amorces unique, mais plusieurs réactions concomitantes d'amplification, de préférence 3, de plusieurs séquences cibles différentes, présentes dans le même échantillon, lesdites réactions mettant en jeu plusieurs couples d'amorces distincts, d'au moins dix et de préférence au moins quinze couples d'amorces.

Ainsi, selon le procédé objet de la présente invention, les séquences d'acides nucléiques cibles sont amplifiées à l'aide de couples d'amorces nucléotidiques spécifiques, l'expression « amorce spécifique » désignant en l'espèce une séquence nucléotidique utile comme amorce pour amplification et capable d'hybrider en conditions strictes avec au moins 80 % des séquences d'acides nucléiques cibles. Les « conditions strictes d'hybridation » obéissent à la définition classique et sont connues de l'homme du métier (21).

Les termes et expressions « amorces », « amorces pour amplification » et « amorces nucléotidiques » sont équivalents au sens de l'invention. Il est entendu que les amorces sont nécessairement spécifiques, même si tel n'est pas toujours explicitement précisé dans le texte.

Le calcul du rapport R, tel qu'il apparaît dans l'étape b) du procédé, consiste en un simple ratio, accessible à l'homme du métier dès lors qu'il se trouve en possession d'un tracé, tel qu'illustré aux figures 1B et 3 et fourni par un séquenceur automatique d'acides nucléiques.

En l'occurrence, un analyseur de séquence, ou séquenceur automatique, du type ABI PRISM® (Perkin-Elmer, Wellesley, MA), détermine une courbe en indiquant en abscisse la longueur en bases des produits d'amplification, et en ordonnée l'intensité de la fluorescence émise par le fluorochrome. La taille des produits d'une part, et le type de fluorochrome associé auxdits produits d'autre part, permettent l'identification des signaux relatifs aux différents marqueurs coamplifiés.

Comme l'illustre la Figure 3, les signaux ou courbes ainsi obtenus se présentent sous la forme de pics plus ou moins hauts.

En cas d'homozygotie, l'on observe un pic unique. En revanche, en cas d'hétérozygotie, deux pics correspondant aux deux allèles présents sont séparés par une distance proportionnelle à la taille du polymorphisme (i.e., la taille de la séquence séparant les deux copies répétées).

Selon un mode de mise en oeuvre avantageux du procédé de détection objet de la présente invention, pour un prélèvement biologique à tester, l'étape a) d'amplification par PCR multiplex est effectuée en double ; et, lors de l'étape b), ce n'est pas le rapport R qui est calculé, mais la moyenne des deux rapports, chacun desdits rapports correspondant à une réaction d'amplification.

Dans l'étape c) du procédé selon l'invention, il est fait référence à un « score statistique global ». Ledit score représente une entité statistique standardisée, permettant d'établir un seuil de significativité (ou seuil de normalité, ou encore seuil de confiance) non empirique, fixe et valable pour tous les individus étudiés. Ainsi est-il possible de conclure à la normalité ou l'anormalité d'un échantillon sur l'ensemble des rapports $R_i$ calculés pour chaque individu, et ce, en se fondant sur des critères fixes, objectifs et indépendants de l'individu considéré. La « globalisation », ou sommation des rapports $R_i$ sur tous les marqueurs testés pour un individu donné, a été dictée par le fait que tous les individus ne possédaient pas les mêmes marqueurs informatifs, ni le même nombre. Or, dans le cadre de l'invention, cet inconvénient se trouve précisément pallié par la quantification d'un DA global, correspondant à un individu, et non plus à un marqueur.

Dans cette étape, le « rapport de référence $R_f$ » dont il est question pour le calcul du « score statistique global » correspond à une moyenne de rapports de hauteurs des pics relatifs aux deux allèles d'un marqueur, lesdits rapports ayant été obtenus à partir d'au moins vingt, et de préférence au moins trente échantillons de référence contenant de l'ADN sain. Ces échantillons de référence peuvent provenir de différents individus, la seule condition qu'ils aient à remplir étant de représenter des échantillons sains.

Les « échantillons de référence », ou « prélèvements de référence », sont des prélèvements biologiques soigneusement choisis comme étant susceptibles de servir de contrôle en ce qu'ils représentent un fluide biologique ou un tissu sain pour les anomalies génétiques recherchées. Ainsi, s'agissant de la détection des TV, le « prélèvement biologique » à tester est d'origine urinaire, tandis que les « échantillons de référence » consistent, par exemple, en des échantillons de sang.

L'on distingue ainsi le « prélèvement biologique » à analyser, susceptible de contenir des cellules tumorales, et les « échantillons de référence », servant de contrôles de normalité, c'est-à-dire comprenant des cellules saines, exemptes d'anomalies génétiques caractéristiques des tumeurs recherchées.

Le « score statistique global » est obtenu d'après la formule suivante :

$$\text{Score} = \Sigma\, d_i^2 = \Sigma\, \{[\text{Ln}R_i - m(\text{Ln}R_f)] \div s(\text{Ln}R_f)\}^2,$$

où:

- $d_i$ désigne une distance statistique, calculée pour un marqueur i ;
- $R_i$ désigne, pour chaque marqueur, le rapport de hauteur des deux pics calculé à partir du prélèvement biologique ;
- $R_f$ désigne, pour chaque marqueur, le rapport de hauteur des deux pics calculé à partir d'un échantillon de référence ;
- $m(\text{Ln}R_f)$ correspond à la moyenne des valeurs de $\text{Ln}R_f$ ; et
- $s(\text{Ln}R_f)$ correspond à l'écart-type des valeurs de $\text{Ln}R_f$.

Conformément à cette formule, le rapport $R_i$ de hauteur des deux pics correspondant aux deux allèles du marqueur i est calculé à partir de l'acide nucléique contenu dans le prélèvement biologique et comparé à la moyenne des rapports de référence $R_f$ correspondant aux échantillons de référence.

Il est entendu que les étapes a) et b) du procédé de détection sont effectuées à la fois pour le prélèvement biologique et les échantillons de référence, de façon à pouvoir établir une comparaison, conformément à l'étape c) dudit procédé.

Afin de rendre la représentation des rapports $R_i$ et $R_f$ symétrique par rapport à 1 (rapport idéal 1/1 pour un individu hétérozygote sain), au sens où il est indifférent que la hauteur du premier pic soit divisée par celle du deuxième ou inversement, dans la mesure où l'entité représentée dans les deux cas correspond à un même écart par rapport à 1 (par exemple, les rapports 1/2 et 2/1 représentent un même écart par rapport à 1/1), les variables étudiées ont été transformées en leur logarithme népérien.

Cette transformation a permis de vérifier que lesdites variables suivaient une loi normale, bien connue des statisticiens.

Une courbe illustrant la loi normale, dite courbe « de Gauss » ou courbe « en cloche » est fournie par la Figure 2 ci-après.

Des moyennes et écarts-type caractéristiques de la loi normale vérifiée par la variable $\text{Ln}R_f$ ont ainsi pu être calculés. Lesdits moyennes et écarts-type sont des valeurs estimées à partir de résultats expérimentaux. Aussi ces valeurs sont-elles respectivement représentées par les lettres latines m et s, et non les lettres grecques $\mu$ et $\sigma$, affectées aux valeurs théoriques de ces mêmes entités statistiques.

Le principe du calcul du score global repose sur celui des distances individuelles pour chaque marqueur, et sommation des carrés de celles-ci.

Une « distance » d correspond, conformément à la formule supra et selon le langage statistique courant, à un écart à la moyenne. En l'espèce, la distance $d_i$ représente l'écart entre $LnR_i$ et $m(LnR_f)$. En tant que différence de lois normales, elle obéit elle-même à une loi normale. Ladite distance est d'autant plus grande que : (i) des anomalies génétiques sont présentes dans le prélèvement biologique ; et (ii) le rapport entre les nombres de cellules tumorales et cellules saines est plus élevé. Afin de standardiser la distance ainsi obtenue, celle-ci est rapportée à la dispersion des mesures de référence pour chaque marqueur, à savoir l'écart-type $s(LnR_f)$.

Le score global $\Sigma\, d_i^2$, donné par la formule supra, en ce qu'il représente la somme des carrés d'une loi normale, est distribué selon une loi du khi-deux à N degrés de liberté, où N représente le nombre total de marqueurs informatifs indépendants testés. En respect des usages dans le domaine statistique, l'on considère que les dénominations « khi-deux », « khi-carré » et « $\chi^2$ » sont équivalentes.

En définitive, c'est au moyen de la loi du khi-deux qu'une borne ou seuil non empirique de significativité ou normalité peut être fixée.

A cette fin, dans un premier temps, le risque de première espèce $\alpha$ est choisi afin de déterminer la spécificité attendue du procédé de détection conforme à l'invention. L'on entend ici par « spécificité », la probabilité que le test soit négatif si les individus sont sains, une telle probabilité étant idéalement égale à 1.

Puis, la distribution du khi-deux, pour cette valeur choisie de $\alpha$ et N degrés de liberté, fournit une valeur limite $\Sigma\, d^2_{max}$, au-delà de laquelle l'on peut conclure, en toute rigueur et objectivité, que le prélèvement biologique étudié est pathologique, en ce qu'il contient effectivement des cellules tumorales.

Selon un autre mode de mise en oeuvre, le procédé de détection objet de la présente invention comprend au moins les étapes suivantes :

a) l'amplification quantitative multiplex desdits marqueurs ;

b) le calcul du rapport R de hauteur des deux pics correspondant aux signaux d'amplification obtenus pour les deux allèles de chaque marqueur ;

e) le calcul d'une distance individuelle $d_i$ pour chaque marqueur i, selon la formule :

$$d_i = [LnR_i - m(LnR_f)] \div s(LnR_f),$$

où :

$R_i$ désigne, pour chaque marqueur, le rapport de hauteur des deux pics calculé à partir du prélèvement biologique ;

$R_f$ désigne, pour chaque marqueur, le rapport de hauteur des deux pics calculé à partir d'un échantillon de référence ;

$m(LnR_f)$ correspond à la moyenne des valeurs de $LnR_f$ ; et

$s(LnR_f)$ correspond à l'écart type des valeurs de $LnR_f$ ; et

f) la comparaison de la valeur de la distance $d_i$ par rapport à l'intervalle de confiance de niveau (1-$\alpha$) correspondant à l'erreur de première espèce $\alpha$ choisie.

Aux fins de la réalisation de la comparaison selon l'étape f) du procédé ci-dessus, une valeur est affectée au risque de première espèce $\alpha$, permettant de définir un intervalle de normalité ou de confiance, pour la valeur de $LnR_i$, à $\alpha$ près. En effet, cet intervalle correspond à (1-$\alpha$) ; il est donc défini à $\alpha$ erreur près. Par exemple, l'intervalle de normalité peut être égal à :

$$m(LnR_f) \pm [3 \times s(LnR_f)],$$

auquel cas $\alpha$ vaut 0,1%.

Selon cet exemple, si l'on obtient une distance individuelle supérieure ou égale à 3 en valeur absolue ($|d_i| \geq 3$), l'on est en mesure de conclure à une anomalie génétique du marqueur i, avec un risque d'erreur inférieur ou égal à 0,1 %.

De manière générale, le procédé de détection selon l'invention est applicable à la recherche des tumeurs, via la détection de DA dans un prélèvement biologique donné, et ce, dès lors que l'on dispose de marqueurs bi-alléliques d'insertion-délétion informatifs et en nombre suffisant pour détecter la plus grande variété possible d'anomalies susceptibles d'être rencontrées dans les cellules tumorales dont la présence est recherchée.

En particulier, ledit procédé peut être appliqué à la détection des tumeurs de la vessie. En ce cas, le prélèvement biologique est constitué d'urines, et le prélèvement de référence, de sang.

Dans le cadre de la détection des tumeurs de la vessie, il est fait usage d'au moins un marqueur choisi parmi les marqueurs représentés dans le tableau III suivant.

**Tableau III**

| Localisation | Marqueur |
|---|---|
| 2q | 919, 1559 |
| 3p | 17,752 |
| 4p | 1983 |
| 4q | 642 |
| 5q | 714, 739, 787 |
| 6q | 402, 440, 471, 627 |
| 8p | 12 |
| 9p | 18, 116, 476, 558, 1410, 1570, 1998, 2065, 2086, 2102 |
| 9q | 118, 289, 475, 516, 561, 682, 1370, 1384, 1583, 1584, 1654, 1921 |
| 10q | 390, 671, 686, 1789 |
| 11p | 1704 |
| 11q | 1982 |
| 13q | 22, 562, 894, 1363, 1698, 1792, 1979 |
| 14q | 1319 |
| 16q | 792, 1187 |
| 17p | 273, 663, 1385, 1657, 1770 |
| 18q | 1697, 1228 |

Dans ce tableau, et comme il ressort de la description détaillée ci-après, l'on entend par « localisation », le bras chromosomique portant le marqueur en question.

Les marqueurs listés dans le Tableau III ont été choisis parmi les séquences des SIDP accessibles via le site http://research.marshfieldclinic.org/, non seulement parce qu'ils couvrent des régions porteuses de gènes d'intérêt, à savoir des oncogènes et gènes suppresseurs de tumeurs, mais également parce qu'ils sont situés au niveau de régions moins ciblées, et sont alors représentatifs de l'instabllité chromosomique globale des TV.

S'agissant de l'amplification de ces marqueurs, le procédé objet de l'invention met préférentiellement en oeuvre au moins un couple d'amorces constitué d'amorces pour amplification spécifiques, dont les séquences SEQ ID Nos. 1 à 134 sont décrites dans le Tableau IV suivant.

**Tableau IV**

| Marqueur | Séquence sens | Seq. ID n° | Séquence antisens | Seq. ID n° | Taille du produit amplifié | Couple d'amorces |
|---|---|---|---|---|---|---|
| 116 | AAGAGGTCTCTGGGCGCTCACACTT | 1 | AAGTACTGAGCATCAGGACTGTATGGGG | 2 | 169/173 | A1 |
| 118 | CCACAGGTGTGCCCATACAGACATT | 3 | CAGGTGAACTGGGTACGCACACTCA | 4 | 138/140 | B1 |
| 17 | TGTATCAGAGGCAATAATTTCCAAAGCAGA | 5 | CGATCCCAGACACTGAAGATGAAATAAGTC | 6 | 136/140 | C1 |
| 18 | GGCTGTTGACTGCACTGGGATTTAGA | 7 | TCATTAACTCTCAGACTGACCTGGGAGC | 8 | 141/144 | D1 |
| 22 | TATGTGTGGCAGTGAAGAGAACAGGTCTTT | 9 | GGGCTATCTTAAGAAATATGAATACTTTGGCT | 10 | 186/192 | E1 |
| 390 | TAATGGTAAACAATATTTTCAGCCACTTTGGA | 11 | CGGATGGGTGGGTATATTTTATTTTCCA | 12 | 171/175 | F1 |
| 402 | AAGAGCCTCTGTTTATGTGGATTGTGGC | 13 | TCTTGGTAAATTGCCATTTTTCATAAAACAA | 14 | 187/191 | G1 |
| 471 | GCTTGCTATCACGGTGTATTGGGCAT | 15 | TGTCAGAAGGGGTTAGTGCTAGTGTTTGA | 16 | 111/114 | H1 |
| 516 | AACTTGCCTGCATTGTACATCATTCCTA | 17 | GGGGATGTTATTATTTCTGAAGTTGGCG | 18 | 97/100 | I1 |
| 561 | CATTGTGGTTACATTAGGGGAAGGCA | 19 | CCCCGACAGTTGTGATGTGTTCGT | 20 | 153/156 | J1 |
| 562 | TGTTTGAGTGCCTTATAAGTTCTGGTTTTCA | 21 | CAGATGTAGCTTTAGGCATTCTTTTTTCTTGT | 22 | 192/194 | K1 |
| 663 | ATATGTTCACTGGCTAAACTATGTGTATCCCA | 23 | CCTGTTTTTGTAGAGCCCTCAAGTTAAGAA | 24 | 126/130 | L1 |
| 671 | ACCATTGGGAATATGTTAAAGAAATTGGCT | 25 | CAGAAAACATCTCATTTTTGACCAGCTACA | 26 | 220/222 | M1 |
| 682 | AAGGTATGAGGAGAGCAGATGCAAAAAG | 27 | GCATGACAAAAATCACTGGGTGGTC | 28 | 193/197 | N1 |
| 686 | AATGGAAAAGTATTTGGTGTTTTTTGAATGTC | 29 | GCCTGACAAAGGTGAAACTCAGTTGAAA | 30 | 210/213 | O1 |
| 714 | GAAAAGCTACATCCCAGTGCTGAAGG | 31 | ATTTAAGGCCACCAGATTGTGAGGAAAC | 32 | 87/89 | P1 |
| 752 | ACTTTCACTAACAAGCCCTTAAACCGAAAA | 33 | TGCCCCATTGTACACCAAAGAATGTTAATA | 34 | 195/197 | Q1 |
| 894 | ATTTCTTTCATTAAGGCTGGGGAGGC | 35 | GGCCACCTCTGAGGATCTGAGCTTTA | 36 | 136/138 | R1 |
| 12 | ACTTTTATTGGCACAGGCATTC | 37 | GAGTTGTTTCTGACCCACTGATCTC | 38 | 129/131 | S1 |
| 22 | TCAAATAAGAGTTGTCATATCCTGCT | 39 | TGAATATGTGTGGCAGTGAAGA | 40 | 128/134 | T1 |
| 116 | TGTATGGGGCTGGCTTTAG | 41 | GCCTCTGAAGAGGTCTCTGG | 42 | 157/161 | U1 |
| 273 | TGTGATCAATTCCAACTGCTG | 43 | AAGGCTTAAAGGAAATCACGTC | 44 | 143/147 | V1 |
| 289 | TTCTTGCAGGCATGAAGCTA | 45 | CTCAACCCCCTTCTCCATAG | 46 | 152/155 | W1 |
| 390 | TTTTCGGATGGGTGGGTAT | 47 | AACAATATTTTCAGCCACTTTGG | 48 | 167/171 | X1 |
| 402 | AGACACCAAAGAGCCTCTGTTTA | 49 | CTTCTCACTAAATTATGTCTTGGTAAATTG | 50 | 208/212 | Y1 |
| 440 | CAATACCCAGCAAAGGATATGG | 51 | GCATCTGTACATAGTAAGCCTACCG | 52 | 105/107 | Z1 |
| 471 | CTTTTTCAAATGCTGCTTGC | 53 | CAGAAGGGGTTAGTGCTAGTGTT | 54 | 122/125 | A2 |
| 475 | GTGCCATTTTGATTCCCATT | 55 | GGCATTCCAGAACCAAAAG | 56 | 215/218 | B2 |
| 476 | GACCTAAAATTGCGGTCATTTC | 57 | GAAAATGCAGGCCTTTCATCA | 58 | 131/134 | C2 |
| 516 | AGTTGGCGCCAGAAATGA | 59 | ATACAAGAGTCCAAGGTAGCCAGT | 60 | 140/143 | D2 |

(suite)

| Marqueur | Séquence sens | Seq. ID n° | Séquence antisens | Seq. ID n° | Taille du produit amplifié | Couple d'amorces |
|---|---|---|---|---|---|---|
| 558 | GGTAGGCATGTAGAAATACGGTTC | 61 | CCAGGATAGCATTCAACAGTTTG | 62 | 145/149 | E2 |
| 561 | CCACTCACTTTCTTGCATGG | 63 | CCCGACAGTTGTGATGTGTT | 64 | 122/125 | F2 |
| 627 | TGTGTTGTTGCTTTGCCTCT | 65 | CATTCCCACGGTTAGCTGTT | 66 | 182/187 | G2 |
| 642 | TTGTGTCTGCCTGTAGTTCAATG | 67 | GTGATTAAACTTGTATTTCCTGAACA | 68 | 114/116 | H2 |
| 714 | TCTTAGGAAAAGCTACATCCCAGT | 69 | TTTAAGGCCACCAGATTGTGA | 70 | 92/94 | I2 |
| 739 | GCTATGTTCAGAAAATGCATCTCACTC | 71 | TGATGTGTGACAGCCAATGAA | 72 | 212/216 | J2 |
| 787 | GCTAGATGGTCCTGTGTCATTG | 73 | GTGATTAATGTGAACTTCCAGTGC | 74 | 137/141 | K2 |
| 792 | TTGCTGTGGCTCTAGGTTGC | 75 | TCGGCAGTTAATGACAGTGATG | 76 | 176/180 | L2 |
| 919 | GGGGATGACAATGAATATGATG | 77 | TGGCATAACACTTAGCAAGCA | 78 | 197/200 | M2 |
| 1187 | TCAGAGACAAGGTCGCTGCT | 79 | TTTCTTCATAGCTACTCCACCACTG | 80 | 141/152 | N2 |
| 1228 | AGCTTCGGAGAATCTATCAAATAGC | 81 | GAACGGGTAAAATGGCAATG | 82 | 204/206 | 02 |
| 1319 | AAACCAGCCAGTTTTCCTGA | 83 | GTACTGGGTAGGTAATACGCTGAGA | 84 | 86/99 | P2 |
| 1363 | GCCAATTACTTTGCCTCTCC | 85 | ACGGGACAAGTCTGTTTGGT | 86 | 154/158 | Q2 |
| 1370 | CAAAAGATGGAGGCTAATATGTTGA | 87 | AATAGTCCATTAGCAAATCCTTCA | 88 | 127/129 | R2 |
| 1384 | GCAGCTTCCAACTGGTTCTT | 89 | GGCTAACCCAGTGAGTCCAA | 90 | 200/204 | S2 |
| 1385 | CTGCTGCAGATTGAACCAA | 91 | AACAGCATCGCTTTAGATACTAGG | 92 | 98/116 | T2 |
| 1410 | GCCCTGAGCCTTTCAAATC | 93 | TGGAACCTCAGTCACACCTAAG | 94 | 86/89 | U2 |
| 1559 | TGTTTCAGGACTGAGCACGA | 95 | CAGGAGGTGTGGCCAGTTT | 96 | 158/161 | V2 |
| 1570 | ATCCTCCCCACTGAACCTC | 97 | GCCCCTCTTCTGCTGGTTAT | 98 | 219/224 | W2 |
| 1583 | CGGGGCGGACAACTAATG | 99 | CAAACAGGACTGAATGAAAACAACA | 100 | 185/190 | X2 |
| 1584 | AGCCACTTGAATTACCTGGAA | 101 | TCTTGGGAAATCGCCTCTC | 102 | 104/106 | Y2 |
| 1654 | CGTAAATGGAGGTTAAATGGCTTC | 103 | CTCCGCACTTATGCTGCAA | 104 | 91/95 | Z2 |
| 1657 | CCCGTTTACACCTGCTGAGT | 105 | CCTGGGGAGTCTAGGTAAGATG | 106 | 214/218 | A3 |
| 1697 | AGTGAGCCAAAATGGACTAAGG | 107 | TAGGGCCTGTCTGACTCCAA | 108 | 223/227 | B3 |
| 1698 | ATTTCCCTCCCAACCCTGT | 109 | GATCAAATTGAAGGACATGAGAGA | 110 | 186/188 | C3 |
| 1704 | CTTTCTCTTCCCATCTTCACTTG | 111 | GCATGCTTAAGGACTGTGAAA | 112 | 221/225 | D3 |
| 1770 | AGAAGAGTGAACGTATTGACATGAG | 113 | GCTTACGGGTTTTCCTCCA | 114 | 113/115 | E3 |
| 1789 | GGAATACAGCATACTCAAATAAAGG | 115 | CCCTGTGCTTAATGTCTGCAA | 116 | 188/191 | F3 |
| 1792 | ATGATGTGGTACCTCTGTCACC | 117 | TACTCTTCCAGGCACTGATAGG | 118 | 79/83 | G3 |
| 1921 | ATCACAGCGGTGAAGCAAAG | 119 | CTTTGGTCAGTAGGGATCCATTT | 120 | 110/114 | H3 |
| 1979 | TGGGTGTCTGAAATGTTAATTGAGC | 121 | GGGTGAGTTCCAGCGTTTCT | 122 | 173/177 | I3 |

(suite)

| Marqueur | Séquence sens | Seq. ID n° | Séquence antisens | Seq. ID n° | Taille du produit amplifié | Couple d'amorces |
|---|---|---|---|---|---|---|
| 1982 | GATATTAAACAAGTAGCATCAGACACAA | 123 | TAGTATGCAGTGGCTGTTGAGA | 124 | 142/146 | J3 |
| 1983 | AGTGGCTCCCTGTGTCTGA | 125 | AATGAGCTTCGTTCTTTGGAC | 126 | 99/101 | K3 |
| 1998 | CTCAACATCTGGACGGAGCA | 127 | AATGGAGTGTGTACTTGTAGAGAGTGA | 128 | 209/213 | L3 |
| 2065 | ACGCCTGGCCTGAAAGTATT | 129 | GTCTGACATCGCCCTCGTAG | 130 | 164/167 | M3 |
| 2086 | AGAAGCAGAATGGGGATGAA | 131 | GGAGTAATAGATTCTGGCATGTG | 132 | 194/210 | N3 |
| 2102 | TGAACTAAAGGTGGGCAGTG | 133 | CAATGAAGCATTTGACAACGTC | 134 | 115/119 | O3 |

Les amorces pour amplification ont été choisies en application du critère suivant, dit de double spécificité, à savoir une spécificité de séquence et de taille.

D'une part, la spécificité de séquence est telle que : (i) lesdites amorces peuvent être utilisées simultanément dans le cadre de l'amplification multiplex conformément au procédé objet de l'invention, et ce, sans risque d'obtenir des produits d'amplification secondaires non désirés susceptibles de fausser l'analyse ; (ii) elles ne sont pas hybridables entre elles, ce qui prévient l'obtention de résultats faussement négatifs ; et (iii) elles sont spécifiques de séquences nucléotidiques elles-mêmes spécifiques de marqueurs donnés.

D'autre part, la spécificité de taille des produits d'amplification générés permet d'attribuer sans ambiguïté, au moyen de méthodes analytiques conventionnelles connues de l'homme du métier, telle l'électrophorèse sur gel, chacun desdits produits au marqueur dont il est issu.

Ce caractère de double spécificité est essentiel, et a été imposé par les conditions de faisabilité technique du procédé objet de l'invention. Convenablement choisies, les amorces pour amplification sont toutes capables d'hybrider de manière spécifique et sélective à des températures d'hybridation voisines, et permettent l'obtention de produits d'amplification dont la taille varie au sein d'un intervalle relativement étroit, par exemple entre 80 et 200 paires de bases (pb), ceci pour faciliter la mise en oeuvre des étapes suivantes du procédé de détection.

Selon un mode de réalisation particulier, l'étape a) d'amplification quantitative multiplex du procédé de détection, des TV notamment, comprend au moins les sous-étapes suivantes :

a1) une étape de dénaturation préalable à 95 °C pendant 7 min ;
a2) une étape de dénaturation à 95 °C pendant 30 secondes ;
a3) une étape d'hybridation à 61 °C pendant 30 secondes ;
a4) une étape d'élongation à 72 °C pendant 30 secondes ;
les étapes a2) à a4) constituant un cycle, ledit cycle étant répété 35 fois ; et
a5) une étape d'élongation finale à 72 °C pendant 10 minutes.

Ces sous-étapes sont préférentiellement, mais non exclusivement, mises en oeuvre lorsque sont utilisées les amorces pour amplification décrites ci-dessus. Lesdites sous-étapes peuvent être également appliquées lors de l'amplification multiplex de SIDP, au moyen d'amorces distinctes de celles citées dans ledit Tableau, dès lors que les exigences évoquées précédemment, quant aux températures d'hybridation desdites amorces notamment, sont respectées.

La présente invention est illustrée par, sans pour autant se limiter aux figures suivantes :

- Figue 1 : exemple de configuration des résultats d'un test de détection d'anomalies génétiques dans un échantillon biologique d'un individu hétérozygote, après traitement par un analyseur de séquence d'acides nucléiques, lesdits acides nucléiques étant marqués à l'aide d'un fluorochrome. Les parties hachurées représentent les surfaces des signaux correspondant aux deux allèles d'un marqueur.

    Figure 1A : cas des microsatellites.
    Figure 1B : cas des SIDP.

- Figure 2 : histogramme représentant les variables $LnR_f$ pour l'ensemble des marqueurs bi-alléliques d'insertion-délétion étudiés dans le cadre de la détection des TV. C'est en réalité la variable $Ln[R_f/m(R_f)]$ qui est ici représentée et ce, afin de supprimer les biais liés à l'amplification par PCR. La distribution des $Ln[R_f/m(R_f)]$ obéit à une loi normale.
- Figure 3 : exemple de tracé fourni par le logiciel GeneScan® 2.1.1 (ABI PRISM®) en utilisant le procédé de détection selon l'invention.

    Figure 3A : tracé en utilisant neuf marqueurs SIDP, parmi lesquels quatre et cinq ont respectivement été marqués à l'aide des fluorochromes HEX et FAM, ainsi qu'un, standard de taille. La taille des produits d'amplification se trouve en abscisse, et l'intensité de la fluorescence en ordonnée. Dans cet exemple, seul deux marqueurs sont hétérozygotes et ont donné lieu à la détection de deux pics.
    Figure 3B : le tableau indique, pour chaque pic, sa durée de migration jusqu'à la fenêtre de lecture, sa taille en nombre de bases, sa hauteur et sa surface.

L'invention sera mieux comprise à l'aide des exemples suivants, donnés à titre purement illustratif. Il est entendu que la présente invention ne se limite en aucune façon auxdits exemples.

## EXEMPLES

Le procédé de détection des anomalies génétiques présentées par les cellules tumorales selon l'invention a été appliqué à la mise en évidence des TV.

Il a été choisi de tester des régions chromosomiques fréquemment décrites dans la littérature comme présentant des DA à l'origine de TV, ceci afin de disposer d'éléments de comparaison. Ainsi, les bras chromosomiques suivants ont été étudiés : 9p, 9q, 17p, 10q, 13q, 6q, 3p et 5q.

### I- Matériels et méthodes

I-1- <u>Patients</u>

Le critère d'inclusion dans l'étude était la découverte d'une TV chez des personnes ne présentant pas d'antécédents de cancer uro-génital.

Les témoins étaient des patients hospitalisés pour divers motifs en urologie (calculs, incontinence urinaire, adénome prostatique ....).

En définitive, seuls ont été exclus les patients et témoins ayant des antécédents de cancer uro-génital.

87 patients ont ainsi été retenus, répartis en 24 témoins et 63 cas. Les caractéristiques histologiques des cas sont récapitulées dans le tableau V suivant.

**Tableau V**

| | *pTa* | *pT1* | *pT2-4* | *Total* |
|---|---|---|---|---|
| *G1* | 9 | 2 | 0 | 11 |
| *G2* | 18 | 6 | 3 | 27 |
| *G3* | 1 | 16 | 8 | 25 |
| *Total* | 28 | 24 | 11 | 63 |

I-2- <u>Marqueurs</u>

Les marqueurs étaient des SIDP dont les séquences sont accessibles sur le site : <u>http://www.research.marshfieidclinic.org</u> .

*I-2-1) Nombre de marqueurs étudiés*

Le taux d'hétérozygotie, et par là-même d'informativité, des microsatellites étant supérieur à celui des SIPD, un nombre plus important de marqueurs SIDP devaient être utilisés pour parvenir au même niveau d'informativité.

C'est pourquoi, tant que faire se pouvait, plusieurs marqueurs ont été sélectionnés sur chaque bras chromosomique retenu. Le bras 9q, en ce qu'il est le siège principal des anomalies dans les TV, a été plus particulièrement étudié, avec quatre marqueurs.

*I-2-2) Choix des loci*

Idéalement, et comme précisé précédemment, les marqueurs devaient non seulement couvrir des régions porteuses d'oncogènes et gènes suppresseurs de tumeurs, mais également des zones moins spécifiques, susceptibles de présenter un DA du fait de l'instabilité chromosomique globale des TV.

Le choix des régions couvertes par les marqueurs a été guidé par la littérature (Tableau III, supra).

I-3- <u>Quantification des solutions d'ADN</u>

Pour chaque patient, l'ADN urinaire et leucocytaire a été extrait des cellules contenues dans le prélèvement biologique et de référence, respectivement, par des méthodes conventionnelles, connues de l'homme du métier.

Les solutions d'ADN, préalablement diluées au 1/100$^{ème}$, ont été marquées avec le fluorochrome PicoGreen® qui se fixe sélectivement à l'ADN double brin. La lecture en fluorescence était effectuée à 520 nm par comparaison à une gamme d'étalonnage sur un fluorimètre. Chaque échantillon a alors été dilué afin d'obtenir une concentration en ADN de 100 ng/$\mu$l. Pour certains échantillons urinaires, la concentration était inférieure à 100 ng/$\mu$l. Les solutions finales ont été conservées à -20°C.

I-4- Amplification par PCR

La première étape consistait en une amplification des différents marqueurs grâce à trois réactions de PCR multiplex, amplifiant chacune six marqueurs.

*I-4-1) L'ADN*

25 ng d'ADN ont été utilisés par PCR. Les réactions ont été mises au point avec 25 ng d'ADN afin d'éviter toute limitation liée à la quantité d'ADN extraite du culot urinaire.

*I-4-2) Les amorces*

Chaque couple d'amorces pour amplification encadrait un polymorphisme d'insertion-délétion, et était marqué, sur l'une seule des amorces, à l'aide d'un fluorochrome FAM ou HEX.

*I-4-3) Le milieu réactionnel*

Il contenait une polymérase (*Taq* Polymérase Gold de Perkin-Elmer à 5 U/μl), un tampon (Buffer 10X de Perkin-Elmer), du $MgCl_2$ (Perkin-Elmer à 25 mM), des désoxynucléotides triphosphates (dATP, dTTP, dGTP et dCTP à 5mM chacun) et de l'eau afin d'obtenir un volume final de 25 μl.

Les Tableaux VI, VII, et VIII ci-dessous indiquent la composition des milieux réactionnels en fonction des couples d'amorces.

**Tableau VI**

| COMPOSANT | TITRE | QUANTITE (μl) |
|---|---|---|
| $MgCl_2$ | 25mM | 2,5 |
| Tampon | 10 X | 2 |
| dNTP | 5 mM x 4 | 1 |
| Taq | 5 U/μl | 0,25 |
| 516 | 10 pmol/μl | 0,9 |
| 116 | 10 pmol/μl | 1,5 |
| 118 | 10 pmol/μl | 1,5 |
| 18 | 10 pmol/μl | 1 |
| 561 | 20 pmol/μl | 1,75 |
| 682 | 50 pmol/μl | 1 |
| ADN | 50 ng | cf. [ADN] |
| $H_2O$ | complément pour obtenir 25μl | |

**Tableau VII**

| COMPOSANT | TITRE | QUANTITE (μl) |
|---|---|---|
| $MgCl_2$ | 25mM | 4 |
| Tampon | 10 X | 2,5 |
| dNTP | 5 mM x 4 | 1 |
| Taq | 5 U/μl | 0,25 |
| 894 | 10 pmol/μl | 0,4 |
| 663 | 10 pmol/μl | 2 |
| 402 | 10 pmol/μl | 2 |
| 22 | 10 pmol/μl | 2 |
| 562 | 10 pmol/μl | 4 |
| ADN | 50 ng | cf. [ADN] |
| $H_2O$ | complément pour obtenir 25μl | |

**Tableau VIII**

| COMPOSANT | TITRE | QUANTITE (µl) |
|---|---|---|
| $MgCl_2$ | 25mM | 3 |
| Tampon | 10 X | 2,5 |
| dNTP | 5 mM X 4 | 1 |
| Taq | 5 U/µl | 0,25 |
| 714 | 10 pmol/µl | 0,3 |
| 471 | 10 pmol/µl | 0,4 |
| 17 | 10 pmol/µl | 0,8 |
| 390 | 10 pmol/µl | 1,6 |
| 752 | 10 pmol/µl | 1,8 |
| 686 | 20 pmol/µl | 2 |
| 671 | 20 pmol/µl | 2 |
| ADN | 50 ng | cf. [ADN] |
| $H_2O$ | complément pour obtenir 25µl | |

*I-4-4) Le programme de PCR*

Identique pour les trois réactions, il comprenait les étapes suivantes :

1. Une phase de dénaturation et d'activation de la polymérase à 95°C pendant 7 minutes
2. Une phase de dénaturation à 95°C pendant 30 secondes
3. Une phase d'hybridation à 61 °C pendant 30 secondes
4. Une phase d'élongation à 72°C pendant 30 secondes Les phases 2-3-4 étaient répétées 35 fois
5. Une élongation finale de 10 minutes à 72°C.

*I-4-5) La mise au point des PCR multiplex*

Chaque couple d'amorces a d'abord été contrôlé individuellement afin de s'assurer de sa réactivité. Les amorces ont ensuite été réunies au sein d'un même mélange réactionnel.

De multiples gammes ont alors été réalisées en jouant sur divers paramètres influant sur la réaction (température d'hybridation, concentration en $MgCl_2$, concentration en polymérase, nombre de cycles d'amplification), ceci afin de déterminer les conditions optimales d'amplification.

En outre, une difficulté majeure était liée au fait que l'amplification des différents produits devait se faire de façon relativement équivalente, afin que les conditions de lecture de l'analyseur de séquences restent valables quel que soit le produit (c'est-à-dire que tous les produits d'amplification soient détectés sans aucune saturation).

I-5- <u>Analyse des fragments amplifiés</u>

Les produits d'amplification ont été séparés en fonction de leur taille, puis quantifiés sur un analyseur ABI PRISM® (modèle 310 à 1 capillaire ou 3100 à 16 capillaires).

Cet appareil fonctionnait comme suit. Les produits d'amplification, marqués par l'amorce fluorescente, ont été mélangés à un tampon dénaturant (formamide) et à un standard de taille interne. Ce mélange a ensuite été dénaturé (chauffage 3 min à 95°C) puis déposé dans l'appareil. Ce dépôt a été chargé électriquement 5 sec à 15 kV, puis a migré dans un capillaire soumis à un champ électrique important (15 kV). Le capillaire était éclairé au niveau d'une fenêtre par un faisceau laser. Le rayonnement lumineux émis par la molécule fluorescente sous l'impact du faisceau laser a été capté par une caméra numérique CDD puis analysé par un logiciel informatique GeneScan®. La quantité de produit présente dans le dépôt était proportionnelle à l'intensité du rayonnement émis. Sa taille a été calculée à partir de la durée de migration du produit lors de l'électrophorèse capillaire jusqu'à la fenêtre du faisceau laser.

Un exemple de tracé est fourni à la Figure 3.

I-6- <u>Détermination d'un déséquilibre allélique</u>

En théorie, un DA se traduit par la modification du rapport 1/1 des deux allèles présents initialement dans la cellule (soit disparition, soit duplication d'un allèle).

Une difficulté était posée par le fait que le culot urinaire contenait des cellules tumorales, mais également des cellules normales provenant de la desquamation de l'urothélium sain. Les proportions respectives de ces deux populations cellulaires étaient variables. Dans ces conditions, un DA se manifestait par des modifications relativement peu sensibles du rapport de hauteur des pics relatifs aux deux allèles. L'importance de ces modifications était proportionnelle au pourcentage de cellules d'origine tumorale dans le culot urinaire et dépendait également du type d'anomalies responsable du DA.

Il était donc nécessaire de définir des critères permettant de décider si une modification du rapport était significative et correspondait effectivement à un DA, ou bien s'il s'agissait d'une différence non significative liée à des variations expérimentales.

Des calculs statistiques ont été pratiqués et sont rapportés dans la partie « II- Résultats » suivante. L'ensemble des résultats a été géré, et les calculs effectués à l'aide du logiciel ACCESS® (Microsoft).

**II- Résultats**

II-1- Mode de calcul des déséquilibres alléliques

Deux modes de calculs différents ont été utilisés.

*II-1-1) Définition de critères propres à chaque marqueur*

La détermination d'un DA reposait sur la comparaison des rapports $R_f$ et $R_i$ de hauteur des deux pics correspondant aux deux allèles du marqueur considéré, respectivement dans le sang et les urines.

Les rapports ici visés ont été rendus symétriques par rapport à 1 en passant en logarithme népérien.

Pour chaque marqueur, les variables $LnR_f$ se sont révélées obéir à une loi normale. En conséquence, une moyenne et un écart-type expérimental des $LnR_f$, appelés respectivement $m(LnR_f)$ et $s(LnR_f)$, ont été déterminés pour chaque marqueur.

Ainsi, en choisissant un risque d'erreur de première espèce $\alpha$ de 0,1 %, un intervalle de normalité a été défini pour la valeur $LnR_i$, comme étant : $LnR_f \pm [3 \times s(LnR_f)]$.

$LnR_f$ étant une variable individuelle aléatoire mesurée expérimentalement, la moyenne m(LnRf) lui a été préférée pour les calculs afin de limiter les fluctuations expérimentales.

La définition de l'intervalle de normalité à 99,9 % était donc :

$$LnR_i \text{ compris entre } [m(LnR_f) \pm 3 \times s(LnR_f)]$$

Le Tableau IX suivant indique, pour chaque marqueur, le nombre d'ADN informatifs (i.e., ADN hétérozygotes) ayant servi à déterminer la distribution des $LnR_f$, $m(LnR_f)$ et $s(LnR_f)$.

**Tableau IX**

| marqueur | nombre d'ADN informatifs | $m(LnR_f)$ | $s(LnR_f)$ |
|---|---|---|---|
| 17 | 34 | 0,0483 | 0,0193 |
| 18 | 38 | 0,0000 | 0,0521 |
| 22 | 55 | 0,0572 | 0,0351 |
| 116 | 39 | 0,1518 | 0,0347 |
| 118 | 39 | -0,1009 | 0,0281 |
| 390 | 44 | 0,0668 | 0,0189 |
| 402 | 53 | 0,0742 | 0,0200 |
| 471 | 46 | 0,0418 | 0,0563 |
| 516 | 49 | 0,0460 | 0,0188 |
| 561 | 40 | -0,0412 | 0,0257 |
| 562 | 42 | 0,0387 | 0,0355 |
| 663 | 43 | 0,0085 | 0,0212 |
| 671 | 36 | 0,0088 | 0,0130 |

(suite)

| marqueur | nombre d'ADN informatifs | $m(LnR_f)$ | $s(LnR_f)$ |
|---|---|---|---|
| 682 | 22 | 0,0767 | 0,0220 |
| 686 | 30 | -0,0289 | 0,0268 |
| 714 | 58 | -0,0106 | 0,0172 |
| 752 | 41 | 0,0217 | 0,0150 |
| 894 | 35 | -0,0093 | 0,0184 |

Comme il est ressort du Tableau IX ci-dessus, $m(LnR_f)$ n'était pas toujours égale à 0 [correspondant à $m(R_f)$ =1], bien qu'il n'existât pas de DA. Cela était dû à des biais d'amplifications, c'est-à-dire l'amplification préférentielle d'un allèle plutôt que l'autre lors de la réaction de PCR.

Ce mode de détermination des DA avait pour inconvénient de reposer sur le cumul des risques de première espèce $\alpha$. En effet, si le risque $\alpha$ était fixe pour un marqueur informatif, il augmentait néanmoins avec le nombre de marqueurs informatifs. Pour pallier cet inconvénient, il fallait déterminer un risque $\alpha$ corrélé au nombre de marqueurs informatifs, ceci afin que le risque $\alpha$ cumulé soit identique d'un patient à l'autre.

Dans cette optique, le calcul d'un score unique prenant en compte l'ensemble des marqueurs testés pour un patient a été mis au point.

*II-1-2) Calcul d'un score global*

Le principe reposait sur le calcul de la distance de $R_i$ aux valeurs moyennes de $R_f$ pour chaque marqueur, d'en faire la somme des carrés après transformation logarithmique et de tester le score ainsi obtenu selon une loi du khi-deux.

Là encore, en raison des fluctuations expérimentales, les hauteurs des pics à partir desquelles étaient calculées $R_i$ et $R_f$ pouvaient être considérées comme des variables aléatoires dont les mesures expérimentales à partir d'une réaction de PCR étaient des estimations.

Pour un résultat expérimental $R_i$ donné, a-t-on pu calculer le carré de la distance de $LnR_i$ à la moyenne m ($LnR_f$) rapporté à l'écart-type $s(LnR_f)$ :

$$d_i^2 = \{ [ LnR_i - m(LnR_f) ] / s(LnR_f) \}^2$$

Pour N marqueurs informatifs, on a établi la somme des carrés des distances appelée score global $\Sigma d_i^2$. Ledit score se distribuait selon une loi du khi-deux à N degrés de liberté. La probabilité que $\Sigma d_i^2$ soit supérieure à une valeur donnée pouvait donc être aisément déterminée à l'aide des abaques.

Une limite supérieure à $\Sigma d_i^2$ a été fixée, correspondant à un risque $\alpha$ donné, déterminant la spécificité du test, pour N marqueurs informatifs. En choisissant par exemple $\alpha$ égal à 5 %, la distribution du khi-deux pour N degrés de libertés permettait de fixer $\Sigma d^2_{max}$, tel que lorsque, pour un échantillon d'urines, l'on obtenait $\Sigma d_i^2$ supérieur à $\Sigma d^2_{max}$, l'on était en mesure de conclure objectivement que les urines considérées étaient pathologiques, avec un risque d'erreur de 5 %.

II-2- Résultats du test

*II-2-1) Résultats globaux*

Pour caractériser les « performances » du procédé de détection selon l'invention et tel que mis en oeuvre dans le cas des TV, l'on définit les paramètres de « sensibilité » et « spécificité » comme suit.

La « sensibilité » correspond à la probabilité que le test soit positif en cas d'anomalie. Idéalement, cette probabilité vaut 1.

La « spécificité », déjà définie précédemment, désigne la probabilité que le test soit négatif en l'absence d'anomalies. Là encore, idéalement, cette probabilité est égale à 1.

S'agissant du calcul par marqueur, il a été défini un intervalle de confiance à 0,1 %. En cas de DA sur un ou plusieurs marqueurs, le test était considéré comme positif. Ce mode de calcul a fourni les résultats suivants : une sensibilité de 76,19 % (48 patients sur 63 présentaient au moins un DA) pour une spécificité de 95 % (1 témoins sur 17 présentait au moins un DA).

La détermination de DA par score a révélé une sensibilité de 68,25 % (43 patients sur 63 présentaient au moins un DA) pour une spécificité de 95 % (1 témoin sur 17 présentait au moins un DA).

*II-2-2) Résultats rapportés aux stade et grade tumoraux*

Le tableau X ci-dessous fournit les sensibilités en fonction du mode de calcul, du stade et du grade histologique.

**Tableau X**

| | n | % de sensibilité (calcul par score) | % de sensibilité (calcul par marqueur) |
|---|---|---|---|
| Global | 63 | 68,25 | 76,19 |
| pTa | 28 | 57,14 | 67,86 |
| pT1 | 24 | 81,82 | 86,37 |
| pT2-4 | 11 | 72,73 | 81,82 |
| G1 | 11 | 54,55 | 63,64 |
| G2 | 27 | 66,67 | 74,07 |
| G3 | 25 | 78,26 | 86,96 |

Cette analyse a mis en évidence une sensibilité moindre pour les lésions pTa par rapport aux pT1 (p = 0,02). L'échantillon de tumeurs pT2-4 était trop peu important pour permettre une comparaison avec les pT1 par le test du khi-deux.

De la même façon, le test était moins sensible pour les lésions G1 que pour les lésions G2 (p = 0,001). Il n'existait pas de différence significative en termes de sensibilité entre les tumeurs G2 et G3 (p > 0,05).

II-3- <u>Intérêt des différents bras chromosomiques</u>

*II-3-1) Sensibilité globale des bras chromosomiques*

Dans le tableau XI suivant, figure pour chaque bras chromosomique la sensibilité observée.

**Tableau XI**

| Bras | 10q | 13q | 3p | 5q | 6q | 9p | 9q | 17p |
|---|---|---|---|---|---|---|---|---|
| Sensibilité | 45 % | 40 % | 34 % | 37 % | 41 % | 42 % | 58 % | 33 % |

*II-3-2) Sensibilité en fonction des bras et des critères histologiques*

Le tableau XII ci-après donne les sensibilités de chaque bras en fonction des stade et grade tumoraux.

**Tableau XII**

| | n | 10q | 13q | 3p | 17p | 5q | 6q | 9p | 9q |
|---|---|---|---|---|---|---|---|---|---|
| pTa | 28 | 33% | 19% | 18% | 8% | 16% | 19% | 47% | 48% |
| pT1 | 24 | 53% | 61% | 38% | 44% | 47% | 58% | 50% | 75% |
| pT2-4 | 11 | 50% | 56% | 29% | 50% | 100% | 40% | 0% | 50% |
| G1 | 11 | 20% | 9% | 0% | 0% | 29% | 38% | 38% | 50% |
| G2 | 27 | 41% | 39% | 25% | 15% | 12% | 25% | 50% | 59% |
| G3 | 25 | 57% | 58% | 42% | 50% | 71% | 52% | 38% | 60% |

II-4- <u>Intérêt des différents marqueurs</u>

La sensibilité a été calculée pour chaque marqueur. Les résultats sont consignés dans le tableau XIII suivant.

**Tableau XIII**

| Marqueur | Sensibilité (%) |
|---|---|
| 17 | 10,53 |
| 18 | 29,63 |
| 22 | 25 |
| 116 | 44 |
| 118 | 44,44 |
| 390 | 40 |
| 402 | 51,61 |
| 471 | 20 |
| 516 | 54,29 |
| 561 | 65,52 |
| 562 | 19,23 |
| 663 | 27,59 |
| 671 | 41,67 |
| 682 | 31,25 |
| 686 | 36,36 |
| 714 | 39,02 |
| 752 | 37,04 |
| 894 | 48,15 |

**III- Conclusions**

III-1- Spécificité du test

La spécificité du test peut être choisie en fonction de la valeur attribuée au risque $\alpha$ (95 % pour le calcul par score dans les expériences ici recensées, c'est-à-dire 1 faux-positif sur 17 témoins).

La spécificité peut être augmentée par l'amélioration de la reproductibilité des réactions d'amplification par PCR. A cet égard, il est avantageux d'effectuer chaque PCR en double. En cas de discordance des résultats, une troisième PCR discriminante peut être alors réalisée.

III-2- Sensibilité du test

Selon le mode de détermination des DA, la sensibilité du test était de 68,25 % (score global) ou 76,19 % (critères individuels de DA).

Une des limites de la sensibilité réside dans le nombre de marqueurs informatifs.

Initialement, ce test avait été conçu pour trente marqueurs dont le taux d'informativité moyen était de 42,62 %. Lesdits marqueurs étaient répartis sur neuf bras chromosomiques. Sept d'entre eux étaient explorés par trois marqueurs chacun, ce qui représentait une probabilité moyenne d'avoir au moins un marqueur informatif sur le bras de 81 %. Les deux autres bras étaient respectivement explorés par sept marqueurs (bras 9p) et deux marqueurs (bras 11p), avec des probabilités d'avoir au moins un marqueur informatif respectivement de 99,98 % et 68 %.

Lors de la première mise au point du test, seuls dix-huit marqueurs ont été retenus, répartis sur huit bras chromosomiques et présentant un taux d'informativité moyen de 43 %.

La probabilité qu'au moins un marqueur soit informatif sur chaque bras chromosomique étudié était en moyenne de 69,5 %.

Au terme de la mise au point du test cinquante-neuf marqueurs ont été retenus, répartis sur dix-sept bras chromosomiques.

La sensibilité a été améliorée par l'utilisation de marqueurs supplémentaires.

III-3- Avantages du test selon l'invention

Du fait de la nature bi-allélique des marqueurs utilisés, les produits d'amplification ont toujours la même taille (taille donnée ± taille du polymorphisme), et le même aspect, à la différence des microsatellites, lesquels donnent lieu à la formation de produits d'amplification multiples et différant les uns des autres par le nombre de répétitions (Figure 1, A et B). Il en résulte, dans le cas du test selon l'invention, une grande facilité de lecture et d'interprétation, ce qui n'est pas le cas avec les microsatellites.

Pour ces mêmes raisons, il s'est avéré possible d'utiliser des protocoles de réactions d'amplification multiplex, lesquelles ne fonctionnent pas avec les microsatellites au-delà de deux, voire trois marqueurs au grand maximum. Cette mise en oeuvre en multiplex a permis de compenser le taux d'informativité des SIPD, plus faible que celui des microsatellites, en augmentant le nombre de marqueurs sans rendre la réalisation trop coûteuse en temps, réactifs, et échantillons.

Enfin, l'observation d'un même profil chez tous les individus hétérozygotes sains a permis de calculer, pour chaque marqueur d'une part, et de manière globale d'autre part, des normes aux fins de la détermination statistiquement rigoureuse et objective de la présence ou non de DA.

III-4- Conclusions

Les DA présentent l'avantage d'être une anomalie spécifique des cellules tumorales, en comparaison d'autres marqueurs connus (télomérases, facteurs de dégradation du fibrinogène, protéine du facteur H...).

L'utilisation de marqueurs de type SIDP permet, après optimisation du test, en particulier augmentation du nombre de marqueurs, d'envisager une spécificité d'environ 95 % et une sensibilité d'environ 80 à 90 %.

En conclusion, le test de dépistage des tumeurs, et en particulier des TV, selon l'invention, en ce qu'il est simple, techniquement fiable et peu coûteux, peut facilement être intégré à l'activité de routine d'un laboratoire de biologie moléculaire.

Ce test non invasif pourrait notamment trouver une indication de choix dans le dépistage des tumeurs urothéliales du haut appareil urinaire, plus difficiles à mettre en évidence que les TV.

**REFERENCES BIBLIOGRAPHIQUES**

(1) Greenlee R.T., Murray T., Bolden S., et al : Cancer statistics, 2000. CA Cancer J. clin.; 50: 7-33.

(2) Boring C., Squires T., Tong T. : Cancer J. clin.; 1995; 45: 8.

(3) Pfister P., Asselain B., Blanchon B., et al: Evolution de l'incidence des cancers déclarés à l'assurance maladie en Ile-de-France entre 1980 et 1994, BEH n° 3, 2000.

(4) Grossman H.B. : New methods for detection of bladder cancer. Semin. Urol. Oncol.; 1998; 16: 17-22.

(5) Ramakumar S., Bhuiyan J., Besse J.A., and al. : Comparison of screening methods in the detection of bladder cancer. J. urol.; 1999; 161: 388-394.

(6) Brown F.M.: Urine cytology, is it still the gold standart for screening? Urol. Clin. of North Am.; Feb. 2000; 27(1): 25-37.

(7) Grocela J. A., McDougal W.S.: Utility of nuclear matrix protein in the detection of bladder reccurent cancer. Urol. Clin. of north Am.; 2000 Feb; 27:47-51.

(8) Lokeshwar V.B., Soloway M.S.: Current bladder tumor tests: does their projected utility fulfill clinical necessity? J. of urology; April 2001; 165:1067-1077.

(9) Malkowicz S.B.: The application of huma complement factor H related protein (BTA TRAK) in minitoring patients with bladder cancer. Urol. Clin. of north Am.; 2000 Feb; 27: 63-73.

(10) Lokeshwar V.B., Block N.L.: HA-Haase urine test: a sensitive and specific method for detecting bladder cancer and evaluating its grade. Urol. Clin. of north Am.; 2000 Feb; 27: 53-61.

(11) Fradet Y., Lockhart C. and al: Performance caracteristics of a new monoclonal antibody test for bladder cancer: Immunocyt. The Canadian Journal of Urology; September 1997; 4(3);400-405.

(12) Bernues M., Casadevall C., Caballin M.R. and al.: Study of allelic losses on 3p, 6q, and 17p in human urothelial cancer. Cancer Genet Cytogenet; 1999 Jul 1; 112(1): 42-5.

(13) Le Duc A., Cussenot O., Desgrandchamps F. : Les tumeurs de vessie, ed. Sanofi-Winthrop, 1994.

(14) Uchida T., Wang C., Wada C. and al.: Microsatellite instability in transitionnal cell carcinoma of the urinary tract and its relationship to clinicopathological variables and smoking. Int J Cancer 69: 142-145, 1996.

(15) Mao L., Lee D.J., Tockman M.S. and al: Microsatellite alterations as clonal markers for the detection of human cancer. Proc. Natl Acad. Sci. USA; 1994; 91: 9871-9875.

(16) Mao L., Schoenberg M.P., Scicchitano M. and al.: Molecular detection of primary bladder cancer by microsatellite analysis. Science; 1996 Feb. 2; 271(5249):659-62.

(17) Baron A., Mastroeni F., Moore P.S. and al.: Detection of bladder cancer by semi automated microsatellite analysis of urine sediment. Adv. Clin. Path.; 2000 Jan; 4(1): 19-24.

(18) Christensen M., Wolf H., Orntoft T.: Microsatellite alterations in urinary sediments from patients with cystitis and bladder cancer. Int. J. Cancer; 2000; 85: 614-617.
(19) Schneider A., Borgnat S., Lang H. and al.: Evaluation of microsatellite analysis in urine sediment for diagnosis of bladder cancer. Cancer Res.; 2000 Aug 15; 60(16): 4617-22.
(20) Steiner G., Schoenberg M.P., Linn J.-F. and al: Detection of bladder reccurence by microsatellite analysis of urine. Nat. Med.; 1997 Jun.; 3(6): 621-4.
(21) Sambrook et Russel. 2001. Molecular cloning: a laboratory manual, 3rd ed., Cold Spring Harbor Laboratory Press, Cold Spring Harbor, N.Y.

## Revendications

**1.** Procédé de détection de cellules tumorales contenues dans un prélèvement biologique par mise en évidence des déséquilibres alléliques d'au moins quinze, et de préférence au moins trente marqueurs chromosomiques bi-alléliques d'insertion-délétion, ledit procédé comprenant au moins les étapes suivantes :

a) l'amplification quantitative multiplex desdits marqueurs ;
b) le calcul du rapport R de hauteur des deux pics correspondant aux signaux d'amplification obtenus pour les deux allèles de chaque marqueur ;
c) le calcul d'un score statistique global, relatif à l'ensemble desdits marqueurs, qui se distribue selon une loi du khi-deux dont le nombre de degrés de liberté est égal au nombre de marqueurs testés, ledit score global étant calculé selon la formule :

$$\text{Score} = \Sigma\, d_i^2 = \Sigma\, \{[\text{LnR}_i - m(\text{LnR}_f)] \div s(\text{LnRf})\}^2,$$

où :

$d_i$ désigne une distance statistique, calculée pour un marqueur i ;
$R_i$ désigne, pour chaque marqueur, le rapport de hauteur des deux pics calculé à partir du prélèvement biologique ;
$R_f$ désigne, pour chaque marqueur, le rapport de hauteur des deux pics calculé à partir d'un échantillon de référence ;
$m(LnR_f)$ correspond à la moyenne des valeurs de $LnR_f$ ; et
$s(LnR_f)$ correspond à l'écart type des valeurs de $LnR_f$ ; et

d) la comparaison dudit score par rapport au seuil de normalité fixé, au-delà duquel le prélèvement biologique est considéré comme pathologique, ledit seuil de normalité correspondant à la valeur du khi-deux pour une valeur choisie du risque de première espèce $\alpha$ et le nombre de marqueurs testés.

**2.** Procédé selon la revendication 1, dans lequel le rapport de référence $R_f$ est une moyenne de rapports de hauteurs des pics relatifs aux deux allèles d'un marqueur, lesdits rapports étant obtenus à partir d'au moins trente échantillons de référence sains.

**3.** Procédé de détection de cellules tumorales contenues dans un prélèvement biologique par mise en évidence des déséquilibres alléliques d'au moins quinze, et de préférence au moins trente marqueurs chromosomiques bi-alléliques d'insertion-déletion, ledit procédé comprenant au moins les étapes suivantes :

a) l'amplification quantitative multiplex desdits marqueurs ;
b) le calcul du rapport R de hauteur des deux pics correspondant aux signaux d'amplification obtenus pour les deux allèles de chaque marqueur ;
e) le calcul d'une distance individuelle $d_i$ pour chaque marqueur i, selon la formule :

$$d_i = [\text{LnR}_i - m(\text{LnR}_f)] \div s(\text{LnRf}),$$

où:

$R_i$ désigne, pour chaque marqueur, le rapport de hauteur des deux pics calculé à partir du prélèvement biologique ;
$R_f$ désigne, pour chaque marqueur, le rapport de hauteur des deux pics calculé à partir d'un échantillon de référence ;
$m(LnR_f)$ correspond à la moyenne des valeurs de $LnR_f$ ; et
$s(LnR_f)$ correspond à l'écart type des valeurs de $LnR_f$ ; et

f) la comparaison de la valeur de la distance $d_i$ par rapport à l'intervalle de confiance de niveau (1-$\alpha$) correspondant à l'erreur de première espèce $\alpha$ choisie.

**4.** Procédé selon la revendication 3, **caractérisé en ce que** l'intervalle de confiance est défini par la formule :

$$m(\text{LnR}_f) \pm [3 \times s(\text{LnR}_f)],$$

où:

Rf désigne, pour chaque marqueur, le rapport de hauteur des deux pics calculé à partir d'un échantillon de référence;
$m(LnR_f)$ correspond à la moyenne des valeurs de LnRf ; et
$s(LnR_f)$ correspond à l'écart-type des valeurs de LnRf.

**5.** Procédé selon l'une quelconque des revendications 1 à 4, **caractérisé en ce que** l'étape d'amplification a) est effectuée en double.

**6.** Procédé selon la revendication 5, dans lequel l'étape b) comprend le calcul de la moyenne des deux rapports R, chacun desdits rapports correspondant à une réaction d'amplification effectuée selon l'étape a).

**7.** Procédé selon l'une quelconque des revendications 1 à 6, **caractérisé en ce que** la spécificité de détection des cellules tumorales est déterminée par la valeur choisie de $\alpha$.

**8.** Procédé selon l'une quelconque des revendications 1 à 7, applicable à la détection des tumeurs de la vessie, dans lequel le prélèvement biologique est d'origine urinaire, et les échantillons de référence d'origine sanguine.

**9.** Procédé selon l'une quelconque des revendications 1 à 8, **caractérisé en ce qu'**au moins un couple d'amorces pour amplification spécifique utilisé dans l'étape a), est constitué d'amorces choisies deux à deux parmi les séquences SEQ ID Nos 1 à 134 de telle sorte que chaque couple soit constitué d'une amorce de SEQ ID No n et d'une amorce de SEQ ID No n+1, où n est un nombre entier impair choisi entre 1 et 133.

**10.** Utilisation des amorces d'amplification spécifiques de marqueurs chromosomiques d'insertion-délétion choisie dans le groupe constitué des séquences SEQ ID Nos 1 à 134, pour la mise en oeuvre d'un procédé selon l'une quelconque des revendications 1 à 9.

## Claims

**1.** Method of detecting tumour cells contained in a biological sample by revealing allelic disequilibria of at least fifteen and preferably at least thirty biallelic chromosomal insertion/deletion markers, said method comprising at least the following steps:

a) multiplex quantitative amplification of said markers;
b) calculation of the height ratio R of the two peaks corresponding to the amplification signals obtained for the two alleles of each marker;
c) calculation of a global statistical score, relating to all said markers, which is distributed according to a chi-square law wherein the number of degrees of freedom is equal to the number of markers tested, said global score being calculated according to the formula:

$$score = \Sigma\, d_i^2 = \Sigma\, \{[LnR_i - m(LnR_f)] + s(LnRf)\}^2,$$

where:

$d_i$ denotes a statistical distance, calculated for a marker i;
$R_i$ denotes, for each marker, the height ratio of the two peaks calculated from the biological sample;
$R_f$ denotes, for each marker, the height ratio of the two peaks calculated from a reference sample;
$m(LnR_f)$ corresponds to the mean of the values of $LnR_f$; and
$s(LnR_f)$ corresponds to the standard deviation of the values of $LnR_f$;

and
d) comparison of said score in relation to the fixed normality threshold, beyond which the biological sample is deemed to be pathological, said normality threshold corresponding to the chi-square value for a chosen first-order risk $\alpha$ and the number of markers tested.

**2.** Method according to claim 1, wherein the reference ratio $R_f$ is a mean of height ratios for the peaks in relation to the two alleles of a marker, said ratios being obtained from at least thirty healthy reference samples.

**3.** Method of detecting tumour cells contained in a biological sample by revealing the allelic disequilibria of at least fifteen and preferably at least thirty biallelic chromosomal insertion/deletion markers, said method comprising at least the following steps:

a) multiplex quantitative amplification of said markers;
b) calculation of the height ratio R of the two peaks corresponding to the amplification signals obtained for the two alleles of each marker;
e) calculation of an individual distance $d_i$ for each marker i, according to the formula:

$$d_i = [LnR_i - m(LnR_f)] + s(LnRf),$$

where:

$R_i$ denotes, for each marker, the height ratio of the two peaks calculated from the biological sample;
$R_f$ denotes, for each marker, the height ratio of the two peaks calculated from a reference sample;
$m(LnR_f)$ corresponds to the mean of the values of $LnR_f$; and
$s(LnR_f)$ corresponds to the standard deviation of the values of $LnR_{f;}$

and
f) comparison of the value of the distance $d_i$ in relation to the confidence interval of level (1-$\alpha$) corresponding to the first order error $\alpha$ chosen.

**4.** Method according to claim 3, **characterised in that** the confidence interval is defined by the formula:

$$m(LnR_f) \pm [3 \text{ x } s(LnR_f)],$$

where:

$R_f$ denotes, for each marker, the height ratio of the two peaks calculated from a reference sample;
$m(LnR_f)$ corresponds to the mean of the values of $LnR_f$; and
$s(LnR_f)$ corresponds to the standard deviation of the values of $LnR_f$.

**5.** Method according to any one of claims 1 to 4, **characterised in that** the amplification step a) is carried out in duplicate.

**6.** Method according to claim 5, wherein step b) comprises calculating the mean of the two ratios R, each of said ratios corresponding to an amplification reaction carried out according to step a).

**7.** Method according to any one of claims 1 to 6, **characterised in that** the specificity of detection of the tumour cells is determined by the chosen value of $\alpha$.

**8.** Method according to any one of claims 1 to 7, which can be applied to the detection of tumours of the bladder, wherein the biological sample originates from the urine, and the reference samples originate from the blood.

**9.** Method according to any one of claims 1 to 8, **characterised in that** at least one pair of primers for specific amplification used in step a) consists of primers selected in twos from the sequences SEQ ID Nos. 1 to 134, such that each pair consists of one primer of SEQ ID No. n and one primer of SEQ ID No. n+1, where n is an odd integer selected between 1 and 133.

**10.** Use of specific amplification primers of chromosomal insertion/deletion markers selected from the group consisting of the sequences SEQ ID Nos. 1 to 134, for carrying out a method according to any one of claims 1 to 9.

**Patentansprüche**

1. Verfahren zur Detektion von in einer biologischen Probe enthaltenen Tumorzellen durch Bestimmung der Allelungleichgewichte von wenigstens fünfzehn und vorzugsweise von wenigstens dreißig biallelischen Insertions-Deletions-Chromosomenmarkern, wobei das Verfahren wenigstens die folgenden Schritte umfasst:

   a) quantitative Multiplexamplifikation der Marker;
   b) Berechnung des Höhenverhältnisses R der zwei Peaks, die den Amplifikationssignalen entsprechen, die für die zwei Allele eines jeden Markers erhalten werden;
   c) Berechnung eines statistischen Gesamtscores bezüglich der Gruppe der Marker, die sich nach dem Gesetz Chi-Quadrat verteilt, wobei die Anzahl der Freiheitsgrade gleich der Anzahl der getesteten Marker ist und wobei sich der Gesamtscore nach der Formel berechnet:

$$Score = \sum d_i^{\,2} = \sum \left\{\left[LnR_i - m\left(LnR_f\right)\right] \div s\left(LnR_f\right)\right\}^2,$$

   wobei:

   $d_i$ den für einen Marker i berechneten statistischen Abstand bezeichnet;
   $R_i$ für jeden Marker das aus einer biologischen Probe berechnete Höhenverhältnis der zwei Peaks bezeichnet;
   $R_f$ für jeden Marker das aus einer Referenzprobe berechnete Höhenverhältnis der zwei Peaks bezeichnet;
   $m(LnR_f)$ dem Mittelwert der $LnR_f$-Werte entspricht; und
   $s(LnR_f)$ der Standardabweichung der $LnR_f$-Werte entspricht; und

   d) Vergleich des Scores mit dem festgesetzten Normwert, über dem die biologische Probe als pathologisch angesehen wird, wobei der Normwert dem Wert von Chi-Quadrat für einen gewählten Risikowert $\alpha$ erster Ordnung und die Anzahl der getesteten Marker entspricht.

2. Verfahren nach Anspruch 1, wobei das Referenzverhältnis $R_f$ ein Mittelwert der Peakhöhenverhältnisse für die zwei Allele eines Markers ist, wobei die Verhältnisse aus wenigstens dreißig gesunden Referenzproben erhalten werden.

3. Verfahren zur Detektion von in einer biologischen Probe enthaltenen Tumorzellen durch Bestimmen der Allelungleichgewichte von wenigstens fünfzehn und vorzugsweise von wenigstens dreißig biallelischen Insertions-Deletions-Chromosomenmarkern, wobei das Verfahren wenigstens die folgenden Schritte umfasst:

   a) quantitative Multiplexamplifikation der Marker;
   b) Berechnung des Höhenverhältnisses R der zwei Peaks, die den Amplifikationssignalen entsprechen, die für die zwei Allele eines jeden Markers erhalten werden;
   e) Berechnung eines individuellen Abstands $d_1$ für jeden Marker i nach der Formel:

$$d_1 = \left[LnR_i - m\left(LnR_f\right)\right] \div s\left(LnR_f\right),$$

   wobei:

   $R_i$ für jeden Marker das aus einer biologischen Probe berechnete
   Höhenverhältnis der zwei Peaks, bezeichnet;
   $R_f$ für jeden Marker das aus der Referenzprobe berechnete Höhenverhältnis der zwei Peaks, bezeichnet;
   $m(LnR_f)$ dem Mittelwert der $LnR_f$-Werte entspricht; und
   $s(LnR_f)$ der Standardabweichung der $LnR_f$-Werte entspricht; und

   f) Vergleich des Abstandswertes $d_1$ mit dem Konfidenzintervall (1-$\alpha$), das dem gewählten Fehler $\alpha$ erster Ordnung entspricht.

4. Verfahren nach Anspruch 3, **dadurch gekennzeichnet, dass** das Konfidenzintervall durch die Formel:

$$m(LnR_f) \pm [3 \cdot s(LnR_f)],$$

definiert ist, wobei:

$R_f$ für jeden Marker das aus der Referenzprobe berechnete Höhenverhältnis der zwei Peaks bezeichnet;
$m(LnR_f)$ dem Mittelwert der $LnR_f$-Werte entspricht; und
$s(LnR_f)$ der Standardabweichung der $LnR_f$-Werte entspricht.

**5.** Verfahren nach einem den Ansprüche 1 bis 4, **dadurch gekennzeichnet, dass** der Amplifikationsschritt a) doppelt ausgeführt wird.

**6.** Verfahren nach Anspruch 5, wobei der Schritt b) die Berechnung des Mittelwertes der beiden Verhältnisse R umfasst, wobei jedes dieser Verhältnisse einer gemäß Schritt a) durchgeführten Amplifikationsreaktion entspricht.

**7.** Verfahren nach einem der Ansprüche 1 bis 6, **dadurch gekennzeichnet, dass** die Spezifität der Tumorzellendetektion durch den für $\alpha$ gewählten Wert bestimmt wird.

**8.** Verfahren nach einem der Ansprüche 1 bis 7, anwendbar zur Detektion von Blasentumoren, wobei die biologische Probe aus Urin und die Referenzproben aus Blut stammen.

**9.** Verfahren nach einem der Ansprüche 1 bis 8, **dadurch gekennzeichnet, dass** wenigstens ein Primerpaar, das für die spezifische Amplifikation in Schritt a) verwendet wird, aus Primern besteht, die paarweise so aus den Sequenzen SEQ ID No 1 bis 134 ausgewählt sind, dass jedes Paar aus einem Primer der SEQ ID No n und einem Primer der SEQ ID No n+1 besteht, wobei n eine ganze ungerade Zahl zwischen 1 und 133 ist.

**10.** Verwendung der für Insertions-Deletions-Chromosomenmarker spezifischen Amplifikationsprimer, ausgewählt aus der Gruppe bestehend aus den Sequenzen SEQ ID No 1 bis 134, zur Durchführung eines Verfahrens nach einem der Ansprüche 1 bis 9.

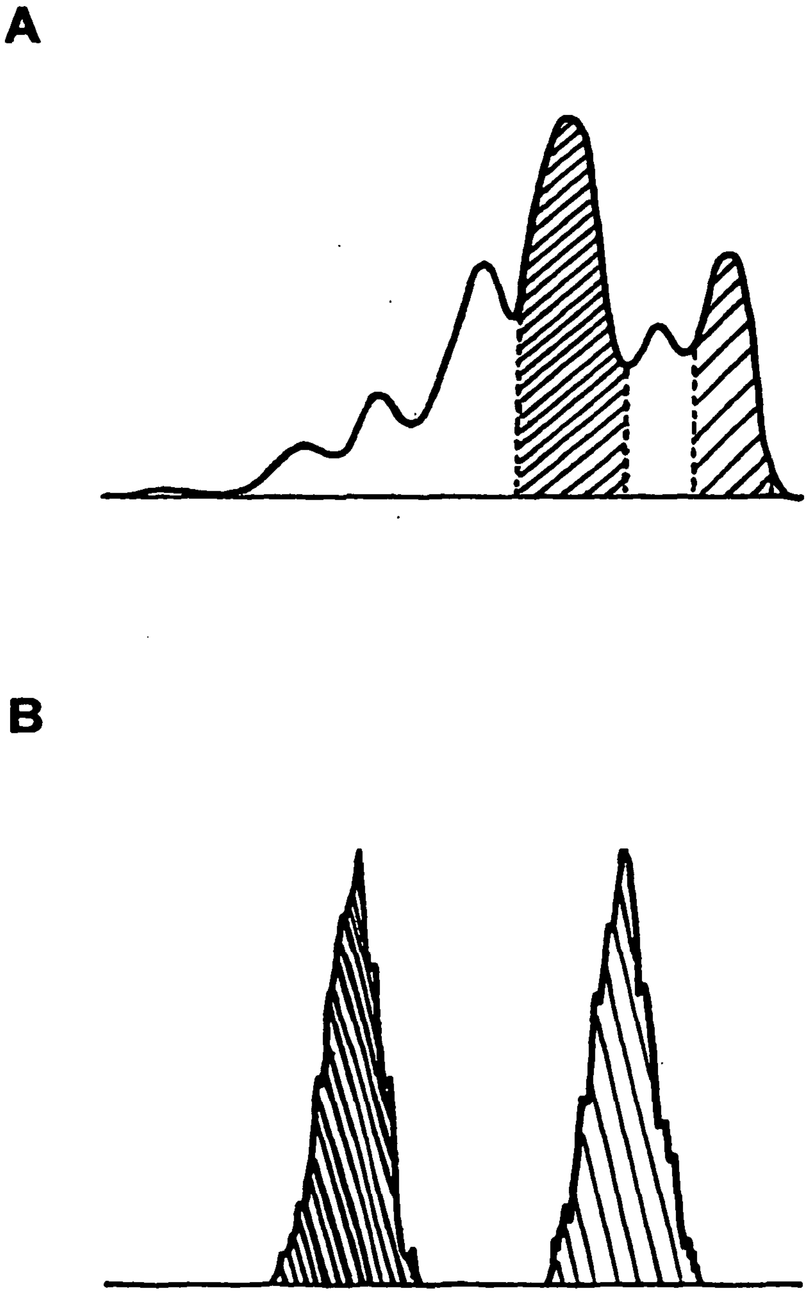


Fig. 1

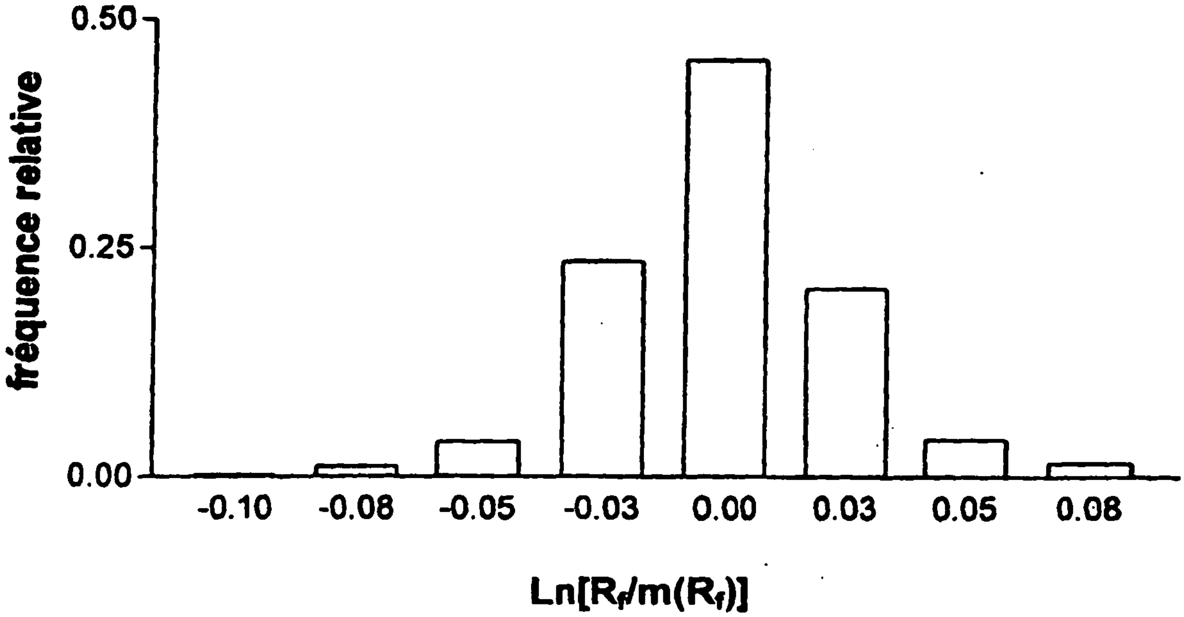
0.50
0.25
0.00
fréquence relative
-0.10
-0.08
-0.05
-0.03
0.00
0.03
0.05
0.08
Ln[Rf/m(Rf)]

Fig. 2

**A**

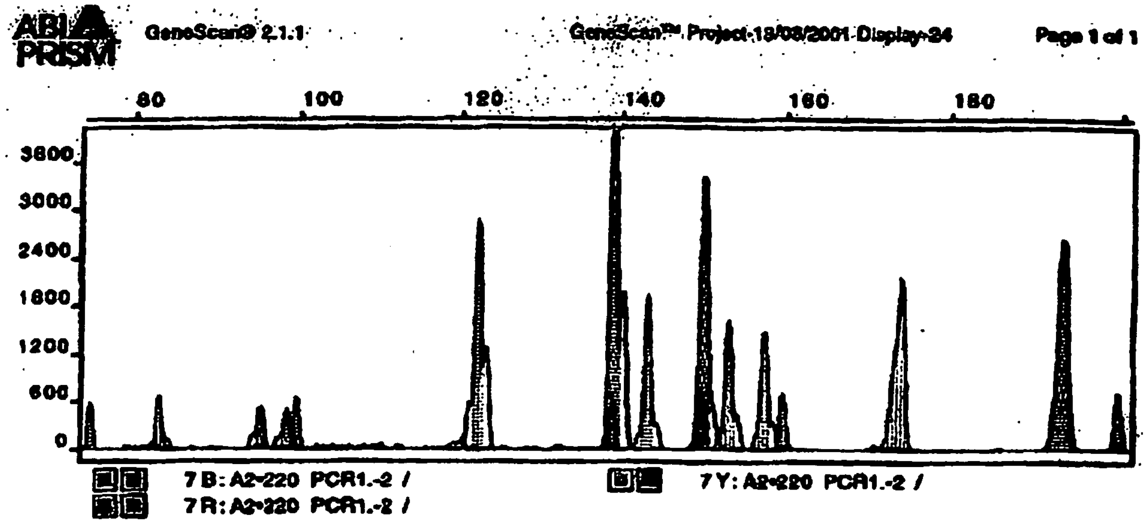


**B**

| Dye/Sample Peak | Minutes | Size | Peak Height | Peak Area | Data Point |
|---|---|---|---|---|---|
| 7B,23 | 18.14 | 83.34 | 710 | 6837 | 4946 |
| 7B,38 | 20.87 | 122.33 | 2902 | 34480 | 5837 |
| 7B,41 | 22.01 | 143.37 | 1844 | 22466 | 6002 |
| 7B,43 | 22.61 | 153.38 | 1848 | 23288 | 6164 |
| 7B,45 | 22.87 | 157.76 | 1487 | 20695 | 6236 |
| 7B,47 | 23.93 | 174.44 | 2183 | 48773 | 6520 |
| 7Y,26 | 18.91 | 95.61 | 559 | 7348 | 5157 |
| 7Y,28 | 19.11 | 98.83 | 516 | 7372 | 5212 |
| 7Y,31 | 21.76 | 139.12 | 4258 | 58939 | 5888 |
| 7Y,33 | 22.42 | 150.31 | 3406 | 49902 | [illegible] |
| 7Y,36 | 25.20 | 193.62 | 2652 | 52672 | [illegible] |
| 7R,3 | 17.81 | 75.00 | 598 | 5864 | [illegible] |
| 7R,4 | 19.19 | 100.00 | 872 | 6408 | [illegible] |
| 7R,5 | 21.75 | 139.00 | 722 | 7439 | 5931 |
| 7R,6 | 22.40 | 150.00 | 725 | 7627 | 6109 |
| 7R,7 | 23.00 | 160.00 | 717 | 7748 | 6273 |
| 7R,8 | 25.83 | 200.00 | 732 | 8851 | [illegible] |

**Fig. 3**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 0300609 W
- FR 0202380


### Littérature non-brevet citée dans la description


- **SCHNEIDER et al.** *Cancer Research,* 2000, vol. 60, 4617-4622
- **GREENLEE R.T ; MURRAY T. ; BOLDEN S et al.** Cancer statistics. *CA Cancer J. clin.,* 2000, vol. 50, 7-33
- **BORING C ; SQUIRES T ; TONG T.** *Cancer J. clin,* 1995, vol. 45, 8
- **PFISTER P ; ASSELAIN B ; BLANCHON B et al.** Evolution de l'incidence des cancers déclarés à l'assurance maladie en Ile-de-France entre 1980 et 1994. *BEH,* 2000
- **GROSSMAN H.B.** New methods for detection of bladder cancer. *Semin. Urol. Oncol.,* 1998, vol. 16, 17-22
- **RAMAKUMAR S ; BHUIYAN J ; BESSE J.A.** Comparison of screening methods in the detection of bladder cancer. *J. urol,* 1999, vol. 161, 388-394
- **BROWN F.M.** Urine cytology, is it still the gold standart for screening?. *Urol. Clin. of North Am,* Février 2000, vol. 27 (1), 25-37
- **GROCELA J. A ; MCDOUGAL W.S.** Utility of nuclear matrix protein in the detection of bladder reccurent cancer. *Urol. Clin. of north Am,* Février 2000, vol. 27, 47-51
- **LOKESHWAR V.B ; SOLOWAY M.S.** Current bladder tumor tests: does their projected utility fulfill clinical necessity?. *J. of urology,* Avril 2001, vol. 165, 1067-1077
- **MALKOWICZ S.B.** The application of huma complement factor H related protein (BTA TRAK) in minitoring patients with bladder cancer. *Urol. Clin. of north Am,* Février 2000, vol. 27, 63-73
- **LOKESHWAR V.B ; BLOCK N.L.** HA-Haase urine test: a sensitive and specific method for detecting bladder cancer and evaluating its grade. *Urol. Clin. of north Am,* Février 2000, vol. 27, 53-61
- **FRADET Y ; LOCKHART C.** Performance caracteristics of a new monoclonal antibody test for bladder cancer: Immunocyt. *The Canadian Journal of Urology,* Septembre 1997, vol. 4 (3), 400-405
- **BERNUES M ; CASADEVALL C ; CABALLIN M.R.** Study of allelic losses on 3p, 6q, and 17p in human urothelial cancer. *Cancer Genet Cytogenet,* 01 Juillet 1999, vol. 112 (1), 42-5
- **LE DUC A ; CUSSENOT O ; DESGRANDCHAMPS F.** Les tumeurs de vessie. 1994
- **UCHIDA T ; WANG C ; WADA C.** Microsatellite instability in transitionnal cell carcinoma of the urinary tract and its relationship to clinicopathological variables and smoking. *Int J Cancer,* 1996, vol. 69, 142-145
- **MAO L ; LEE D.J ; TOCKMAN M.S.** Microsatellite alterations as clonal markers for the detection of human cancer. *Proc. Natl Acad. Sci. USA,* 1994, vol. 91, 9871-9875
- **MAO L ; SCHOENBERG M.P ; SCICCHITANO M.** Molecular detection of primary bladder cancer by microsatellite analysis. *Science,* 02 Février 1996, vol. 271 (5249), 659-62
- **BARON A ; MASTROENI F ; MOORE P.S.** Detection of bladder cancer by semi automated microsatellite analysis of urine sediment. *Adv. Clin. Path,* Janvier 2000, vol. 4 (1), 19-24
- **CHRISTENSEN M ; WOLF H ; ORNTOFT T.** Microsatellite alterations in urinary sediments from patients with cystitis and bladder cancer. *Int. J. Cancer,* 2000, vol. 85, 614-617
- **SCHNEIDER A ; BORGNAT S ; LANG H.** Evaluation of microsatellite analysis in urine sediment for diagnosis of bladder cancer. *Cancer Res.,* 15 Août 2000, vol. 60 (16), 4617-22
- **STEINER G ; SCHOENBERG M.P ; LINN J.-F.** Detection of bladder reccurence by microsatellite analysis of urine. *Nat. Med,* Juin 1997, vol. 3 (6), 621-4
- **SAMBROOK ; RUSSEL.** Molecular cloning: a laboratory manual. Cold Spring Harbor Laboratory Press, 2001

LISTE DE SEQUENCES

<110> ASSISTANCE PUBLIQUE / HOPITAUX DE PARIS
<120> PROCEDE DE DETECTION IN VITRO DES CANCERS PAR LA MISE EN EVIDENCE DE DESEQUILIBRES ALLELIQUES DE MARQUEURS D'INSERTION-DELETION.
<130> B5162A _AD/DBE/CAL
<140> PCT/FR 03/00609
<141> 2003-02-25
<150> FR 0202380
<151> 2002-02-25
<160> 134
<170> PatentIn Ver. 2.1
<210> 1
<211> 25
<212> ADN
<213> Séquence artificielle
<220>
<223> Description de la séquence artificielle: amorce
<400> 1
aagaggtctc tgggcgctca cactt 25
<210> 2
<211> 28
<212> ADN
<213> Séquence artificielle
<220>
<223> Description de la séquence artificielle: amorce
<400> 2
aagtactgag catcaggact gtatgggg 28
<210> 3
<211> 25
<212> ADN
<213> Séquence artificielle
<220>
<223> Description de la séquence artificielle: amorce
<400> 3
ccacaggtgt gcccatacag acatt 25
<210> 4
<211> 25
<212> ADN
<213> Séquence artificielle
<220>
<223> Description de la séquence artificielle: amorce
<400> 4
caggtgaact gggtacgcac actca 25
<210> 5
<211> 30
<212> ADN
<213> Séquence artificielle

<220>
<223> Description de la séquence artificielle: amorce
<400> 5
tgtatcagag gcaataattt ccaaagcaga 30
<210> 6
<211> 30
<212> ADN
<213> Séquence artificielle
<220>
<223> Description de la séquence artificielle: amorce
<400> 6
cgatcccaga cactgaagat gaaataagtc 30
<210> 7
<211> 26
<212> ADN
<213> Séquence artificielle
<220>
<223> Description de la séquence artificielle: amorce
<400> 7
ggctgttgac tgcactggga tttaga 26
<210> 8
<211> 28
<212> ADN
<213> Séquence artificielle
<220>
<223> Description de la séquence artificielle: amorce
<400> 8
tcattaactc tcagactgac ctgggagc 28
<210> 9
<211> 30
<212> ADN
<213> Séquence artificielle
<220>
<223> Description de la séquence artificielle: amorce
<400> 9
tatgtgtggc agtgaagaga acaggtcttt 30
<210> 10
<211> 32
<212> ADN
<213> Séquence artificielle
<220>
<223> Description de la séquence artificielle: amorce
<400> 10
gggctatctt aagaaatatg aatactttgg ct 32
<210> 11
<211> 32
<212> ADN
<213> Séquence artificielle
<220>
<223> Description de la séquence artificielle: amorce
<400> 11
taatggtaaa caatattttc agccactttg ga 32
<210> 12
<211> 28
<212> ADN
<213> Séquence artificielle
<220>
<223> Description de la séquence artificielle: amorce

<400> 12
cggatgggtg ggtatatttt attttcca 28
<210> 13
<211> 28
<212> ADN
<213> Séquence artificielle
<220>
<223> Description de la séquence artificielle: amorce
<400> 13
aagagcctct gtttatgtgg attgtggc 28
<210> 14
<211> 31
<212> ADN
<213> Séquence artificielle
<220>
<223> Description de la séquence artificielle: amorce
<400> 14
tcttggtaaa ttgccatttt tcataaaaca a 31
<210> 15
<211> 26
<212> ADN
<213> Séquence artificielle
<220>
<223> Description de la séquence artificielle: amorce
<400> 15
gcttgctatc acggtgtatt gggcat 26
<210> 16
<211> 29
<212> ADN
<213> Séquence artificielle
<220>
<223> Description de la séquence artificielle: amorce
<400> 16
tgtcagaagg ggttagtgct agtgtttga 29
<210> 17
<211> 28
<212> ADN
<213> Séquence artificielle
<220>
<223> Description de la séquence artificielle: amorce
<400> 17
aacttgcctg cattgtacat cattccta 28
<210> 18
<211> 28
<212> ADN
<213> Séquence artificielle
<220>
<223> Description de la séquence artificielle: amorce
<400> 18
ggggatgtta ttatttctga agttggcg 28
<210> 19
<211> 26
<212> ADN
<213> Séquence artificielle
<220>
<223> Description de la séquence artificielle: amorce
<400> 19
cattgtggtt acattagggg aaggca 26

<210> 20
<211> 24
<212> ADN
<213> Séquence artificielle
<220>
<223> Description de la séquence artificielle: amorce
<400> 20
ccccgacagt tgtgatgtgt tcgt 24
<210> 21
<211> 31
<212> ADN
<213> Séquence artificielle
<220>
<223> Description de la séquence artificielle: amorce
<400> 21
tgtttgagtg ccttataagt tctggttttc a 31
<210> 22
<211> 32
<212> ADN
<213> Séquence artificielle
<220>
<223> Description de la séquence artificielle: amorce
<400> 22
cagatgtagc tttaggcatt cttttttctt gt 32
<210> 23
<211> 32
<212> ADN
<213> Séquence artificielle
<220>
<223> Description de la séquence artificielle: amorce
<400> 23
atatgttcac tggctaaact atgtgtatcc ca 32
<210> 24
<211> 30
<212> ADN
<213> Séquence artificielle
<220>
<223> Description de la séquence artificielle: amorce
<400> 24
cctgtttttg tagagccctc aagttaagaa 30
<210> 25
<211> 30
<212> ADN
<213> Séquence artificielle
<220>
<223> Description de la séquence artificielle: amorce
<400> 25
accattggga atatgttaaa gaaattggct 30
<210> 26
<211> 30
<212> ADN
<213> Séquence artificielle
<220>
<223> Description de la séquence artificielle: amorce
<400> 26
cagaaaacat ctcatttttg accagctaca 30
<210> 27
<211> 28

<212> ADN
<213> Séquence artificielle
<220>
<223> Description de la séquence artificielle: amorce
<400> 27
aaggtatgag gagagcagat gcaaaaag 28
<210> 28
<211> 25
<212> ADN
<213> Séquence artificielle
<220>
<223> Description de la séquence artificielle: amorce
<400> 28
gcatgacaaa aatcactggg tggtc 25
<210> 29
<211> 32
<212> ADN
<213> Séquence artificielle
<220>
<223> Description de la séquence artificielle: amorce
<400> 29
aatggaaaag tatttggtgt tttttgaatg tc 32
<210> 30
<211> 28
<212> ADN
<213> Séquence artificielle
<220>
<223> Description de la séquence artificielle: amorce
<400> 30
gcctgacaaa ggtgaaactc agttgaaa 28
<210> 31
<211> 26
<212> ADN
<213> Séquence artificielle
<220>
<223> Description de la séquence artificielle: amorce
<400> 31
gaaaagctac atcccagtgc tgaagg 26
<210> 32
<211> 28
<212> ADN
<213> Séquence artificielle
<220>
<223> Description de la séquence artificielle: amorce
<400> 32
atttaaggcc accagattgt gaggaaac 28
<210> 33
<211> 30
<212> ADN
<213> Séquence artificielle
<220>
<223> Description de la séquence artificielle: amorce
<400> 33
actttcacta acaagccctt aaaccgaaaa 30
<210> 34
<211> 30
<212> ADN
<213> Séquence artificielle

<220>
<223> Description de la séquence artificielle: amorce
<400> 34
tgccccattg tacaccaaag aatgttaata 30
<210> 35
<211> 26
<212> ADN
<213> Séquence artificielle
<220>
<223> Description de la séquence artificielle: amorce
<400> 35
atttctttca ttaaggctgg ggaggc 26
<210> 36
<211> 26
<212> ADN
<213> Séquence artificielle
<220>
<223> Description de la séquence artificielle: amorce
<400> 36
ggccacctct gaggatctga gcttta 26
<210> 37
<211> 22
<212> ADN
<213> Séquence artificielle
<220>
<223> Description de la séquence artificielle: amorce
<400> 37
acttttattg gcacaggcat tc 22
<210> 38
<211> 25
<212> ADN
<213> Séquence artificielle
<220>
<223> Description de la séquence artificielle: amorce
<400> 38
gagttgtttc tgacccactg atctc 25
<210> 39
<211> 26
<212> ADN
<213> Séquence artificielle
<220>
<223> Description de la séquence artificielle: amorce
<400> 39
tcaaataaga gttgtcatat cctgct 26
<210> 40
<211> 22
<212> ADN
<213> Séquence artificielle
<220>
<223> Description de la séquence artificielle: amorce
<400> 40
tgaatatgtg tggcagtgaa ga 22
<210> 41
<211> 19
<212> ADN
<213> Séquence artificielle
<220>
<223> Description de la séquence artificielle: amorce

<400> 41
tgtatggggc tggctttag 19
<210> 42
<211> 20
<212> ADN
<213> Séquence artificielle
<220>
<223> Description de la séquence artificielle: amorce
<400> 42
gcctctgaag aggtctctgg 20
<210> 43
<211> 21
<212> ADN
<213> Séquence artificielle
<220>
<223> Description de la séquence artificielle: amorce
<400> 43
tgtgatcaat tccaactgct g 21
<210> 44
<211> 22
<212> ADN
<213> Séquence artificielle
<220>
<223> Description de la séquence artificielle: amorce
<400> 44
aaggcttaaa ggaaatcacg tc 22
<210> 45
<211> 20
<212> ADN
<213> Séquence artificielle
<220>
<223> Description de la séquence artificielle: amorce
<400> 45
ttcttgcagg catgaagcta 20
<210> 46
<211> 20
<212> ADN
<213> Séquence artificielle
<220>
<223> Description de la séquence artificielle: amorce
<400> 46
ctcaaccccc ttctccatag 20
<210> 47
<211> 19
<212> ADN
<213> Séquence artificielle
<220>
<223> Description de la séquence artificielle: amorce
<400> 47
ttttcggatg ggtgggtat 19
<210> 48
<211> 23
<212> ADN
<213> Séquence artificielle
<220>
<223> Description de la séquence artificielle: amorce
<400> 48
aacaatattt tcagccactt tgg 23

<210> 49
<211> 23
<212> ADN
<213> Séquence artificielle
<220>
<223> Description de la séquence artificielle: amorce
<400> 49
agacaccaaa gagcctctgt tta 23
<210> 50
<211> 30
<212> ADN
<213> Séquence artificielle
<220>
<223> Description de la séquence artificielle: amorce
<400> 50
cttctcacta aattatgtct tggtaaattg 30
<210> 51
<211> 22
<212> ADN
<213> Séquence artificielle
<220>
<223> Description de la séquence artificielle: amorce
<400> 51
caatacccag caaaggatat gg 22
<210> 52
<211> 25
<212> ADN
<213> Séquence artificielle
<220>
<223> Description de la séquence artificielle: amorce
<400> 52
gcatctgtac atagtaagcc taccg 25
<210> 53
<211> 20
<212> ADN
<213> Séquence artificielle
<220>
<223> Description de la séquence artificielle: amorce
<400> 53
ctttttcaaa tgctgcttgc 20
<210> 54
<211> 23
<212> ADN
<213> Séquence artificielle
<220>
<223> Description de la séquence artificielle: amorce
<400> 54
cagaaggggt tagtgctagt gtt 23
<210> 55
<211> 20
<212> ADN
<213> Séquence artificielle
<220>
<223> Description de la séquence artificielle: amorce
<400> 55
gtgccatttt gattcccatt 20
<210> 56
<211> 19

<212> ADN
<213> Séquence artificielle
<220>
<223> Description de la séquence artificielle: amorce
<400> 56
ggcattccag aaccaaaag 19
<210> 57
<211> 22
<212> ADN
<213> Séquence artificielle
<220>
<223> Description de la séquence artificielle: amorce
<400> 57
gacctaaaat tgcggtcatt tc 22
<210> 58
<211> 21
<212> ADN
<213> Séquence artificielle
<220>
<223> Description de la séquence artificielle: amorce
<400> 58
gaaaatgcag gcctttcatc a 21
<210> 59
<211> 18
<212> ADN
<213> Séquence artificielle
<220>
<223> Description de la séquence artificielle: amorce
<400> 59
agttggcgcc agaaatga 18
<210> 60
<211> 24
<212> ADN
<213> Séquence artificielle
<220>
<223> Description de la séquence artificielle: amorce
<400> 60
atacaagagt ccaaggtagc cagt 24
<210> 61
<211> 24
<212> ADN
<213> Séquence artificielle
<220>
<223> Description de la séquence artificielle: amorce
<400> 61
ggtaggcatg tagaaatacg gttc 24
<210> 62
<211> 23
<212> ADN
<213> Séquence artificielle
<220>
<223> Description de la séquence artificielle: amorce
<400> 62
ccaggatagc attcaacagt ttg 23
<210> 63
<211> 20
<212> ADN
<213> Séquence artificielle

<220>
<223> Description de la séquence artificielle: amorce
<400> 63
ccactcactt tcttgcatgg 20
<210> 64
<211> 20
<212> ADN
<213> Séquence artificielle
<220>
<223> Description de la séquence artificielle: amorce
<400> 64
cccgacagtt gtgatgtgtt 20
<210> 65
<211> 20
<212> ADN
<213> Séquence artificielle
<220>
<223> Description de la séquence artificielle: amorce
<400> 65
tgtgttgttg ctttgcctct 20
<210> 66
<211> 20
<212> ADN
<213> Séquence artificielle
<220>
<223> Description de la séquence artificielle: amorce
<400> 66
cattcccacg gttagctgtt 20
<210> 67
<211> 23
<212> ADN
<213> Séquence artificielle
<220>
<223> Description de la séquence artificielle: amorce
<400> 67
ttgtgtctgc ctgtagttca atg 23
<210> 68
<211> 26
<212> ADN
<213> Séquence artificielle
<220>
<223> Description de la séquence artificielle: armoce
<400> 68
gtgattaaac ttgtatttcc tgaaca 26
<210> 69
<211> 24
<212> ADN
<213> Séquence artificielle
<220>
<223> Description de la séquence artificielle: armoce
<400> 69
tcttaggaaa agctacatcc cagt 24
<210> 70
<211> 21
<212> ADN
<213> Séquence artificielle
<220>
<223> Description de la séquence artificielle: amorce

<400> 70
tttaaggcca ccagattgtg a 21
<210> 71
<211> 27
<212> ADN
<213> Séquence artificielle
<220>
<223> Description de la séquence artificielle: amorce
<400> 71
gctatgttca gaaaatgcat ctcactc 27
<210> 72
<211> 21
<212> ADN
<213> Séquence artificielle
<220>
<223> Description de la séquence artificielle: amorce
<400> 72
tgatgtgtga cagccaatga a 21
<210> 73
<211> 22
<212> ADN
<213> Séquence artificielle
<220>
<223> Description de la séquence artificielle: amorce
<400> 73
gctagatggt cctgtgtcat tg 22
<210> 74
<211> 24
<212> ADN
<213> Séquence artificielle
<220>
<223> Description de la séquence artificielle: amorce
<400> 74
gtgattaatg tgaacttcca gtgc 24
<210> 75
<211> 20
<212> ADN
<213> Séquence artificielle
<220>
<223> Description de la séquence artificielle: amorce
<400> 75
ttgctgtggc tctaggttgc 20
<210> 76
<211> 22
<212> ADN
<213> Séquence artificielle
<220>
<223> Description de la séquence artificielle: amorce
<400> 76
tcggcagtta atgacagtga tg 22
<210> 77
<211> 22
<212> ADN
<213> Séquence artificielle
<220>
<223> Description de la séquence artificielle: amorce
<400> 77
ggggatgaca atgaatatga tg 22

<210> 78
<211> 21
<212> ADN
<213> Séquence artificielle
<220>
<223> Description de la séquence artificielle: amorce
<400> 78
tggcataaca cttagcaagc a 21
<210> 79
<211> 20
<212> ADN
<213> Séquence artificielle
<220>
<223> Description de la séquence artificielle: amorce
<400> 79
tcagagacaa ggtcgctgct 20
<210> 80
<211> 25
<212> ADN
<213> Séquence artificielle
<220>
<223> Description de la séquence artificielle: amorce
<400> 80
tttcttcata gctactccac cactg 25
<210> 81
<211> 25
<212> ADN
<213> Séquence artificielle
<220>
<223> Description de la séquence artificielle: amorce
<400> 81
agcttcggag aatctatcaa atagc 25
<210> 82
<211> 20
<212> ADN
<213> Séquence artificielle
<220>
<223> Description de la séquence artificielle: amorce
<400> 82
gaacgggtaa aatggcaatg 20
<210> 83
<211> 20
<212> ADN
<213> Séquence artificielle
<220>
<223> Description de la séquence artificielle: amorce
<400> 83
aaaccagcca gttttcctga 20
<210> 84
<211> 25
<212> ADN
<213> Séquence artificielle
<220>
<223> Description de la séquence artificielle: amorce
<400> 84
gtactgggta ggtaatacgc tgaga 25
<210> 85
<211> 20

<212> ADN
<213> Séquence artificielle
<220>
<223> Description de la séquence artificielle: amorce
<400> 85
gccaattact ttgcctctcc 20
<210> 86
<211> 20
<212> ADN
<213> Séquence artificielle
<220>
<223> Description de la séquence artificielle: amorce
<400> 86
acgggacaag tctgtttggt 20
<210> 87
<211> 25
<212> ADN
<213> Séquence artificielle
<220>
<223> Description de la séquence artificielle: amorce
<400> 87
caaaagatgg aggctaatat gttga 25
<210> 88
<211> 24
<212> ADN
<213> Séquence artificielle
<220>
<223> Description de la séquence artificielle: amorce
<400> 88
aatagtccat tagcaaatcc ttca 24
<210> 89
<211> 20
<212> ADN
<213> Séquence artificielle
<220>
<223> Description de la séquence artificielle: amorce
<400> 89
gcagcttcca actggttctt 20
<210> 90
<211> 20
<212> ADN
<213> Séquence artificielle
<220>
<223> Description de la séquence artificielle: amorce
<400> 90
ggctaaccca gtgagtccaa 20
<210> 91
<211> 19
<212> ADN
<213> Séquence artificielle
<220>
<223> Description de la séquence artificielle: amorce
<400> 91
ctgctgcaga ttgaaccaa 19
<210> 92
<211> 24
<212> ADN
<213> Séquence artificielle

<220>
<223> Description de la séquence artificielle: amorce
<400> 92
aacagcatcg ctttagatac tagg 24
<210> 93
<211> 19
<212> ADN
<213> Séquence artificielle
<220>
<223> Description de la séquence artificielle: amorce
<400> 93
gccctgagcc tttcaaatc 19
<210> 94
<211> 22
<212> ADN
<213> Séquence artificielle
<220>
<223> Description de la séquence artificielle: amorce
<400> 94
tggaacctca gtcacaccta ag 22
<210> 95
<211> 20
<212> ADN
<213> Séquence artificielle
<220>
<223> Description de la séquence artificielle: amorce
<400> 95
tgtttcagga ctgagcacga 20
<210> 96
<211> 19
<212> ADN
<213> Séquence artificielle
<220>
<223> Description de la séquence artificielle: amorce
<400> 96
caggaggtgt ggccagttt 19
<210> 97
<211> 19
<212> ADN
<213> Séquence artificielle
<220>
<223> Description de la séquence artificielle: amorce
<400> 97
atcctcccca ctgaacctc 19
<210> 98
<211> 20
<212> ADN
<213> Séquence artificielle
<220>
<223> Description de la séquence artificielle: amorce
<400> 98
gcccctcttc tgctggttat 20
<210> 99
<211> 18
<212> ADN
<213> Séquence artificielle
<220>
<223> Description de la séquence artificielle: amorce

<400> 99
cggggcggac aactaatg 8
<210> 100
<211> 25
<212> ADN
<213> Séquence artificielle
<220>
<223> Description de la séquence artificielle: amorce
<400> 100
caaacaggac tgaatgaaaa caaca 25
<210> 101
<211> 21
<212> ADN
<213> Séquence artificielle
<220>
<223> Description de la séquence artificielle: amorce
<400> 101
agccacttga attacctgga a 21
<210> 102
<211> 19
<212> ADN
<213> Séquence artificielle
<220>
<223> Description de la séquence artificielle: amorce
<400> 102
tcttgggaaa tcgcctctc 19
<210> 103
<211> 24
<212> ADN
<213> Séquence artificielle
<220>
<223> Description de la séquence artificielle: amorce
<400> 103
cgtaaatgga ggttaaatgg cttc 24
<210> 104
<211> 19
<212> ADN
<213> Séquence artificielle
<220>
<223> Description de la séquence artificielle: amorce
<400> 104
ctccgcactt atgctgcaa 19
<210> 105
<211> 20
<212> ADN
<213> Séquence artificielle
<220>
<223> Description de la séquence artificielle: amorce
<400> 105
cccgtttaca cctgctgagt 20
<210> 106
<211> 22
<212> ADN
<213> Séquence artificielle
<220>
<223> Description de la séquence artificielle: amorce
<400> 106
cctggggagt ctaggtaaga tg 22

<210> 107
<211> 22
<212> ADN
<213> Séquence artificielle
<220>
<223> Description de la séquence artificielle: amorce
<400> 107
agtgagccaa aatggactaa gg 22
<210> 108
<211> 20
<212> ADN
<213> Séquence artificielle
<220>
<223> Description de la séquence artificielle: amorce
<400> 108
tagggcctgt ctgactccaa 20
<210> 109
<211> 19
<212> ADN
<213> Séquence artificielle
<220>
<223> Description de la séquence artificielle: amorce
<400> 109
atttccctcc caaccctgt 19
<210> 110
<211> 24
<212> ADN
<213> Séquence artificielle
<220>
<223> Description de la séquence artificielle: amorce
<400> 110
gatcaaattg aaggacatga gaga 24
<210> 111
<211> 23
<212> ADN
<213> Séquence artificielle
<220>
<223> Description de la séquence artificielle: amorce
<400> 111
ctttctcttc ccatcttcac ttg 23
<210> 112
<211> 21
<212> ADN
<213> Séquence artificielle
<220>
<223> Description de la séquence artificielle: amorce
<400> 112
gcatgcttaa ggactgtgaa a 21
<210> 113
<211> 25
<212> ADN
<213> Séquence artificielle
<220>
<223> Description de la séquence artificielle: amorce
<400> 113
agaagagtga acgtattgac atgag 25
<210> 114
<211> 19

<212> ADN
<213> Séquence artificielle
<220>
<223> Description de la séquence artificielle: amorce
<400> 114
gcttacgggt tttcctcca 19
<210> 115
<211> 25
<212> ADN
<213> Séquence artificielle
<220>
<223> Description de la séquence artificielle: amorce
<400> 115
ggaatacagc atactcaaat aaagg 25
<210> 116
<211> 21
<212> ADN
<213> Séquence artificielle
<220>
<223> Description de la séquence artificielle: amorce
<400> 116
ccctgtgctt aatgtctgca a 21
<210> 117
<211> 22
<212> ADN
<213> Séquence artificielle
<220>
<223> Description de la séquence artificielle: amorce
<400> 117
atgatgtggt acctctgtca cc 22
<210> 118
<211> 22
<212> ADN
<213> Séquence artificielle
<220>
<223> Description de la séquence artificielle: amorce
<400> 118
tactcttcca ggcactgata gg 22
<210> 119
<211> 20
<212> ADN
<213> Séquence artificielle
<220>
<223> Description de la séquence artificielle: amorce
<400> 119
atcacagcgg tgaagcaaag 20
<210> 120
<211> 23
<212> ADN
<213> Séquence artificielle
<220>
<223> Description de la séquence artificielle: amorce
<400> 120
ctttggtcag tagggatcca ttt 23
<210> 121
<211> 25
<212> ADN
<213> Séquence artificielle

<220>
<223> Description de la séquence artificielle: amorce
<400> 121
tgggtgtctg aaatgttaat tgagc 25
<210> 122
<211> 20
<212> ADN
<213> Séquence artificielle
<220>
<223> Description de la séquence artificielle: amorce
<400> 122
gggtgagttc cagcgtttct 20
<210> 123
<211> 28
<212> ADN
<213> Séquence artificielle
<220>
<223> Description de la séquence artificielle: amorce
<400> 123
gatattaaac aagtagcatc agacacaa 28
<210> 124
<211> 22
<212> ADN
<213> Séquence artificielle
<220>
<223> Description de la séquence artificielle: amorce
<400> 124
tagtatgcag tggctgttga ga 22
<210> 125
<211> 19
<212> ADN
<213> Séquence artificielle
<220>
<223> Description de la séquence artificielle: amorce
<400> 125
agtggctccc tgtgtctga 19
<210> 126
<211> 21
<212> ADN
<213> Séquence artificielle
<220>
<223> Description de la séquence artificielle: amorce
<400> 126
aatgagcttc gttctttgga c 21
<210> 127
<211> 20
<212> ADN
<213> Séquence artificielle
<220>
<223> Description de la séquence artificielle: amorce
<400> 127
ctcaacatct ggacggagca 20
<210> 128
<211> 27
<212> ADN
<213> Séquence artificielle
<220>
<223> Description de la séquence artificielle: amorce

<400> 128
aatggagtgt gtacttgtag agagtga 27
<210> 129
<211> 20
<212> ADN
<213> Séquence artificielle
<220>
<223> Description de la séquence artificielle: amorce
<400> 129
acgcctggcc tgaaagtatt 20
<210> 130
<211> 20
<212> ADN
<213> Séquence artificielle
<220>
<223> Description de la séquence artificielle: amorce
<400> 130
gtctgacatc gccctcgtag 20
<210> 131
<211> 20
<212> ADN
<213> Séquence artificielle
<220>
<223> Description de la séquence artificielle: amorce
<400> 131
agaagcagaa tggggatgaa 20
<210> 132
<211> 23
<212> ADN
<213> Séquence artificielle
<220>
<223> Description de la séquence artificielle: amorce
<400> 132
ggagtaatag attctggcat gtg 23
<210> 133
<211> 20
<212> ADN
<213> Séquence artificielle
<220>
<223> Description de la séquence artificielle: amorce
<400> 133
tgaactaaag gtgggcagtg 20
<210> 134
<211> 22
<212> ADN
<213> Séquence artificielle
<220>
<223> Description de la séquence artificielle: amorce
<400> 134
caatgaagca tttgacaacg tc 22